# EUROPEAN PATENT SPECIFICATION

(11) **EP 1 755 616 B1**
(45) Date of publication and mention of the grant of the patent: **14.08.2013**
(21) Application number: 05735924.2
(22) Date of filing: 08.04.2005
(51) Int. Cl.: A61K 31/573, A61K 31/56, A61P 27/02

(54) **Treatment of exudative retinopathy with mineralcorticoids**
Behandlung von exsudativer Retinopathie mit Mineralcorticoiden
Traitement de la rétinopathie exsudative avec des corticoides mineraux

(30) Priority: 08.04.2004 AU 2004901916; 03.09.2004 AU 2004905034; 03.09.2004 AU 2004905035
(43) Date of publication of application: 28.02.2007
(73) Proprietor: Advanced Ocular Systems Limited, Nedlands, Western Australia 6009 (AU)
(72) Inventor: PENFOLD, Philip Leslie, Aranda, ACT 2614 (AU); PEYMAN, Gholam Ali, New Orleans, LA 70124 (US); SANDERS, Donald Robert, Elmhurst, IL 60126 (US)
(74) Representative: Harding, Charles Thomas
(86) International application number: PCT/US2005/012103
(87) International publication number: WO 2005/099715

(56) References cited:
- WO-A-00/25771
- WO-A-87/07141
- WO-A-2004/073602
- WO-A-2004/073607
- WO-A-2005/082374
- US-A- 6 011 023
- US-B1- 6 395 294
- DATABASE WPI Section Ch, Week 200280 Derwent Publications Ltd., London, GB; Class B05, AN 2002-738968 XP002365232 & RU 2 188 021 C2 (MIKROKHIRURGIYA GLAZA SCI TECH COMPLEX) 27 August 2002 (2002-08-27)
- TOMMILA P ET AL: "Cortisone, heparin and argon laser in the treatment of corneal neovascularization." ACTA OPHTHALMOLOGICA. SUPPLEMENT. 1987, vol. 182, 1987, pages 89-92, XP008059247
- DATABASE WPI Section Ch, Week 200344 Derwent Publications Ltd., London, GB; Class B05, AN 2003-464683 XP002365716 & RU 2 193 399 C2 (MIKROKHIRURGIYA GLAZA SCI TECH COMPLEX) 27 November 2002 (2002-11-27)
- GEORGE S P ET AL: "Inhibition of corneal neovascularization with beta cyclodextrin and hydrocortisone" INVESTIGATIVE OPHTHALMOLOGY AND VISUAL SCIENCE, vol. 36, no. 4, 1995, page S31, XP008059234 & ANNUAL MEETING OF THE ASSOCIATION FOR RESEARCH IN VISION AND OPHTHALMOLOGY; FORT LAUDERDALE, FLORIDA, USA; MAY 14-19, 1995 ISSN: 0146-0404
- VILLEGAS DEL CUVILLO G: "[Experimental study of conjunctivitis treated with hydrocortisone, butazolidin and camoquin.]" ARCHIVOS DEL INSTITUTO DE FARMACOLOGÍA EXPERIMENTAL (MEDICINA). 1961, vol. 13, 1961, pages 77-90, XP008059248 ISSN: 0024-9629
- EL-GAMMAL M Y ET AL: "The role of cortisone and its derivatives in corneal graft rejection." BULLETIN OF THE OPHTHALMOLOGICAL SOCIETY OF EGYPT. 1970, vol. 63, no. 67, 1970, pages 97-110, XP008059244 ISSN: 0078-5342
- ZOLOG N ET AL: "CORTISONE THERAPY AND RELAPSES IN UVEITIS" ARCHIVES DE L'UNION MEDICALE BALKANIQUE, vol. 8, no. 3, 1970, pages 303-308, XP008059261
- REDDY V M ET AL: "EFFECTS OF SODIUM BUTYRATE AND HYDROCORTISONE HC ON HUMAN Y-79 RETINOBLASTOMA CELLS IN CULTURE" INVESTIGATIVE OPHTHALMOLOGY AND VISUAL SCIENCE, vol. 29, no. ABSTR. ISSUE, 1988, page 340, XP008059265 & ANNUAL SPRING MEETING OF THE ASSOCIATION FOR RESEARCH IN VISION AND OPHTHALMOLOGY, SARASOTA, FLORIDA ISSN: 0146-0404
- VOTOCKOVA J: "[VERNAL CONJUNCTIVITIS AND CORTISONE THERAPY.]" CESKOSLOVENSKÁ OFTALMOLOGIE. MAY 1964, vol. 20, May 1964 (1964-05), pages 177-180, XP008059271 ISSN: 0009-059X
- KIMURA R ET AL: "Effect of orally administered hydrocortisone on the ocular tension in primary open-angle glaucoma subjects. Preliminary report." ACTA OPHTHALMOLOGICA. AUG 1976, vol. 54, no. 4, August 1976 (1976-08), pages 430-436, XP008059270 ISSN: 0001-639X
- SOEFKER A ET AL: "Complications of intraocular injections of crystalline cortisone as treatment of progressive exudative age-related macular degeneration" IOVS, vol. 42, no. 4, 15 March 2001 (2001-03-15), page S232, XP008059262 & ANNUAL MEETING OF THE ASSOCIATION FOR RESEARCH IN VISION AND OPHTHALMOLOGY; FORT LAUDERDALE, FLORIDA, USA; APRIL 29-MAY 04, 2001

## Description

This application claims benefit of Australian Provisional Patent Application No. 2004901916 filed 8 April 2004, Australian Provisional Patent Application No. 2004905034 filed 3 September 2004, and Australian Provisional Patent Application No. 2004905035 filed 3 September 2004.

### Field of the Invention

The present description is directed to the administration of therapeutic compounds for the treatment of ophthalmic conditions. The description is also directed to the administration of therapeutic compounds that modulate the activity of mineralocorticoid receptors to treat, ameliorate or prevent ophthalmic conditions. More particularly, the description is directed to the treatment of ophthalmic conditions in the posterior region of the retina with an anti-oedematous steroid. The description also relates to a method of manufacturing a medicament for treatment of ophthalmic conditions.

### Background

Ophthalmic conditions may comprise front-of-eye diseases such as, for example, corneal oedema, anterior uveitis, pterygium, corneal diseases or opacifications with an exudative or inflammatory component, conjunctivitis, allergy and laser induced exudation or back-of-eye diseases such as, for example, exudative macular degeneration, macular oedema arising from laser treatment of the retina, diabetic retinopathy, age-related macular degeneration or retinopathy of prematurity. Back-of-eye diseases comprise the largest number of causes for vision loss in the developed world.

Conjunctivitis is inflammation of the thin mucus membrane that lines the inside of the eyelids and extends over the front of the sclera, often due to. This inflammation is often due to bacterial or viral infection or allergy. Most conjunctivitis manifests as a pale watery swelling or oedema of the conjunctiva and sometimes the whole eyelid, often with a non-puss containing mucoid discharge. Tenderness and oedema around the eye has also been reported.

Corneal oedema is the accumulation of fluid or swelling in the cornea, which can produce clouding of the cornea and significant vision occlusion. Corneal oedema may occur following surgery on the eye, LASIK, LASEK or laser therapy (particularly cataract surgery) or may occur spontaneously, with inflammation of the cornea. When the cornea swells, it thickens and becomes cloudy, reducing the passage of light reaching into the eye.

Anterior uveitis is the inflammation of uveal tissues (the iris and ciliary body). The exact cause of anterior uveitis is unknown, but it may be associated with certain systemic diseases. The general clinical signs of uveitis include: flare (proteins and/or cells apparent in the anterior chamber, resulting from breakdown in the blood-aqueous barrier), redness or hyperemia of the episcleral/scleral blood vessels, extreme sensitivity to light and keratitic precipitates.

The pathogenesis of exudative retinopathies involves blood-retinal barrier (BRB) compromise and inflammation. The retina is principally formed of neuronal matter, and the barrier between the retina and the complex and vigorous vascular system of the choroid behind the retina is very similar to the blood-brain barrier. The BRB is formed at two principal sites: an inner barrier consisting of retinal vascular endothelial cells that line the blood vessels of the choroid; and an outer barrier consisting of the retinal pigment epithelial (RPE) cells that divide the choroid from the retina. Functionally the BRB is dependant on the integrity of the RPE, the retinal vasculature and associated glial limitations, a sheath of cellular processes that inhibit the direct access of blood vessels to the neuronal environment. The BRB functions to preserve the physiological environment of the neuronal retina.

When the BRB is compromised, leakage of fluids from the blood system across the BRB into the retina can cause problems such as exudative retinopathies and vision impairment. Ailments associated with breakdown of the BRB in the posterior region of the retina include, for example, oedematous retinal conditions such as, myopic retinopathies, macular oedema such as clinical macular oedema or angiographic cystoid macular oedema arising from various aetiologies such as diabetes arising from various aetiologies, exudative macular degeneration and macular oedema arising from laser treatment of the retina.

Myopic retinopathy is a condition that results from severe malformation of the retina in part due to overgrowth of the sclera. The deformation leads to restriction of the blood vessels of the choroid, and subsequently abnormal growth of vessels in an attempt to compensate. The new abnormal vessels are fragile and prone to leakage and exudation, leading to exudative retinopathy.

Macular oedema such as clinical macular oedema or angiographic cystoid macular oedema is a condition involving swelling of the macula and typically occurs as a result of aetiologies such as disease (eg diabetes), injury or more rarely, eye surgery. Fluid collects within the layers of the macula, causing blurred, distorted central vision.

In exudative macular degeneration (also known as "wet" or neovascular age-related macular degeneration (wet-AMD)) abnormal overgrowth of blood vessels from the choroid into the retina occurs, compromising the BRB. The abnormal blood vessels are fragile and prone to leakage. Treatment of such diseases currently focuses on removing or inhibiting vascular growth by laser treatment, drug therapy or a combination of both.

Among the angiogenic retinal diseases, diabetic retinopathy is the leading cause of blindness in adults between the ages of 18 to 72. Histological studies consistently implicate endothelial cell dysfunction in the pathology. Diabetic retinopathy is recognised as one of the diabetic microangiopathies, which are severe complications of diabetes. In the initial stage, capillary microaneurysm and dot haemorrhage are observed. Thereafter, cotton wool patches resulting from microvascular obstruction, and retinal oedema, hard exudates or the like resulting from vascular hyperpermeability are observed and neovascularization appears. In the last stage, retinal detachment is caused by the traction of connective tissues grown in the vitreous body. Further, iris rubeosis and neovascular glaucoma are caused, leading to blindness.

Retinal ischemia or degeneration is another disease state that is quite common. It may be produced by injury, tumours or the like, or be associated with various disorders such as where occlusion of a blood vessel or elevated intraocular pressure reduces availability of blood, oxygen or other nutrients to the retina or optic nerve which can result in neuronal cell death (degeneration) and loss of vision. Such disorders include e.g. diabetes, atherosclerosis, venous capillary insufficiency, obstructive arterial and venous retinopathies, glaucoma, and senile macular degeneration. Optic nerve injury and damage also can result in vision loss and can arise from a variety of conditions or incidents.

Although the underlying cause of these angiogenic retinal diseases might be different, the factors initiating the angiogenic process are similar. One of the most important factors in the initiation of an angiogenic response is the up-regulation of the expression of vascular endothelial growth factor (VEGF), a potent angiogenic and permeability factor, the up-regulation of which has been shown in patients suffering from neovascular eye diseases.

The most widely used treatment for these disorders is retinal laser photocoagulation, an ablative treatment that destroys the peripheral retina to preserve the central macula, which is responsible for 80% of human vision. A wide range of theories have been proposed to explain the beneficial effects of retinal laser photocoagulation in delaying retinal angiogenesis, however, the underlying molecular mechanism remains to be elucidated.

Laser therapy is directed to removal or blockage of blood vessels via photodynamic therapy or laser photocoagulation. For example, focal laser treatment may be applied to microaneurysms identified in diabetic retinopathy. The therapeutic effects of laser photocoagulation are thought to be due to the destruction of photoreceptors, the highest oxygen consumers in the retina. Subsequently, these photoreceptors are replaced by glial cells allowing increased oxygen diffusion from the choroid to the inner retina thereby relieving inner retinal hypoxia. This improved oxygenation triggers a two-pronged cascade of events where: (1) constriction of the retinal arteries results in decreased hydrostatic pressure in capillaries and the constriction of capillaries and venules; and (2) the cellular production of VEGF is inhibited. Together, these effects are believed to ultimately result in the inhibition of neovascularization and a decrease in oedema. Cell proliferation and regulation of cellular proteins are induced by the laser photocoagulation, and their therapeutic effect might be an essential part of the physiological response.

However, a complication of laser treatment is inflammation, leading to further oedema. In addition, laser treatment is not always a permanent cure as the blood vessels may begin to grow again, and microaneurysms may reform. Furthermore, laser treatment of abnormal blood vessels cannot be performed on vessels located in certain regions of the retina, such as the central region of the macula.

Pharmaceutical interventions with drug compounds having anti-angiogenic or angiostatic properties, such as anecortave acetate or anti-vascular endothelial growth factor (VEGF) compounds (Lucentis^{®}, Macugen^{®}), have also been proposed as methods for treatment. However, to date there is insufficient evidence to indicate how successful these compounds will be.

The present invention seeks to provide an improved method for using a therapeutically effective amount of a compound capable of modulating the activity of mineralocorticoid receptors within cells or tissue located in an eye or adjacent to an eye for the manufacture of a composition for the prophylactic or therapeutic treatment of an individual with a ophthalmic condition wherein said ophthalmic condition is exudative retinopathy, wherein the treatment of the ophthalmic conditions at least slows the rate of development of the ophthalmic condition. More particularly the invention seeks to provide a method of manufacture of a medicament for the treatment of ophthalmic conditions such as exudative eye diseases and specifically exudative retinopathy, which method addresses the principal problem underlying the diseases (eg exudation of fluids from vessels).

Citation or identification of any document in this application is not an admission that such a document is available as prior art to the present invention.

### Summary of the Invention

The present description provides a composition for the treatment of an individual with an ophthalmic condition comprising the step of: administering to the individual a therapeutically effective amount of a compound capable of modulating the activity of mineralocorticoid receptors within cells or tissue located in an eye or adjacent to an eye in the individual to be treated. Preferably, said compound is an anti-oedematous steroid, more preferably a mineralocorticoid.

Also provided is a composition for the prophylactic or therapeutic treatment of exudative retinopathies in a patient comprising the step of: administering a therapeutically effective amount of a compound capable of modulating the activity of mineralocorticoid receptors to or adjacent to cells or tissue from or in said patient that are afflicted with an exudative retinopathy; and wherein said cells or tissue are selected from the group consisting of: retinal cells, retinal pigment epithelial cells, the epithelial cells of the BRB, choroidal endothelial cells.

The descriction further provides a composition for the prophylactically treating an individual with an ophthalmic condition in a first eye but not a second eye comprising the steps of: administering to the second eye of said individual a therapeutically effective amount of a compound capable of modulating the activity of mineralocorticoid receptors within cells or tissue located in or adjacent to the eye.

In a first preferred aspect, the description provides a composition for the treatment of an individual with an ophthalmic condition comprising the step of: administering to the individual a therapeutically effective amount of a compound capable of modulating the activity of mineralocorticoid receptors within cells or tissue located in an eye or adjacent to an eye wherein said compound is a mineralocorticoid of the general structure: 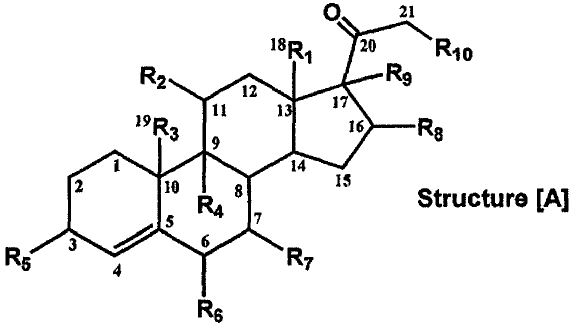 wherein
R₁ is CH₃, CH=O, CH₂OH, CR=O, CH₂NH₂, COCHCH₂, NO₂ X or CH₂X wherein X is F, Cl, Br;
R₂ is H, OH, =O, NH₂ or CN;
R₃ is H, CH₃ or CH₂OH;
R₄ is H, F or Cl;
R₅ is H, OH, =O, SH, NH₂, CN, NO₂, CH₃, CH₂OH, CH=O, X, CH₂X, OR, OCOR, OPO(OR)₂, =CH₂ or CHR wherein X is F, Cl, Br;
R₆ is H, CH₃ or X wherein X is F, Cl, Br;
R₇ is H, OH, CH₃, Alkyl, Ph, X, OCH₃, OR, SCOCH₃, SCOR, OCOR, CH₂OH, CH₂X, CH=O, CR=O or NHCOR wherein X is F, Cl, Br;
R₈ is H, OH, CH₃, CH₂CH₃, SH, NH₂ or X wherein X is F, Cl, Br;
R₉ is H, OH, SH, NH₂, CH₃, CH₂CH₃ or X wherein X is F, Cl, Br;
R₁₀ is H, OH, CH₃, CH₂CH₃, SH, NH₂, CH₂OH, CH₂CH₂OH, OR, OCOR, OPO(OR)₂, NHCOR CH=O, X or CH₂X wherein X is F, Cl, Br; and
wherein said compound is not a compound disclosed in United States Patent 6,011,023 where the compound is to be used to treat or prevent ocular diseases selected from the list comprising retinal diseases (diabetic retinopathy, chronic glaucoma, retinal detachment, sickle cell retinopathy, senile macular degeneration due to subretinal neovascularization), rubeosis iritis, inflammatory diseases, chronic uveitis, neoplasms (retinoblastoma, pseudoglioma), Fuchs' heterochromic iridocyclitis, neovascular glaucoma, corneal neovascularization (inflammatory, transplantation, developmental hypoplasia of the iris, corneal graft neovascuiarization), neovascularization following a combined vitrectomy and lensectomy, vascular diseases (retinal ischemia, choroidal vascular insufficiency, choroidal thrombosis, carotid artery ischemia), pterygium, neovascularization of the optic nerve, neovascularization due to penetration of the eye or contusive ocular injury, and retrolental fibroplasia.

In a second preferred aspect, the description provides a treatment of an individual with an ophthalmic condition comprising the step of: administering to the individual a therapeutically effective amount of a compound capable of modulating the activity of mineralocorticoid receptors within cells or tissue located in an eye or adjacent to an eye; wherein said compound is a mineralocorticoid of the general structure: 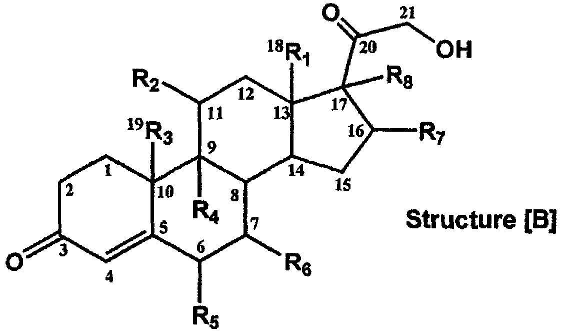 wherein
R₁ is CH₃, CH=O or COCHCH₂;
R₂ is H, OH or =O;
R₃ is H, CH₃ or CH₂OH;
R₄ is H, or F;
R₅ R₆ and R₇ are H;
R₈ is H or OH; and
wherein said compound is not a compound disclosed in United States Patent 6,011,023 where the compound is to be used to treat or prevent ocular diseases selected from the list comprising retinal diseases (diabetic retinopathy, chronic glaucoma, retinal detachment, sickle cell retinopathy, senile macular degeneration due to subretinal neovascularization), rubeosis iritis, inflammatory diseases, chronic uveitis, neoplasms (retinoblastoma, pseudoglioma), Fuchs' heterochromic iridocyclitis, neovascular glaucoma, corneal neovascularization (inflammatory, transplantation, developmental hypoplasia of the iris, corneal graft neovascularization), neovascularization following a combined vitrectomy and lensectomy, vascular diseases (retinal ischemia, choroidal vascular insufficiency, choroidal thrombosis, carotid artery ischemia), pterygium, neovascularization of the optic nerve, neovascularization due to penetration of the eye or contusive ocular injury, and retrolental fibroplasia.

In a third preferred aspect, the description provides a treatment of an individual with an ophthalmic condition comprising the step of: administering to the individual a therapeutically effective amount of a compound capable of modulating the activity of mineralocorticoid receptors within cells or tissue located in an eye or adjacent to an eye wherein said compound is a mineralocorticoid of the general structure: 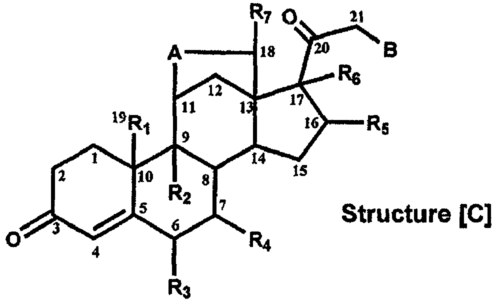 wherein
A is O, S, NH, CH₂, CHOH, C=O, CHX, CHCH₃, CH₂CH₂, OCH₂, SCH₂, NHCH₂, NRCH₂, NCOR wherein X is F, Cl, Br;
B is H, OH, SH, NH₂, CH₃, CH₂OH, CH₂CH₂OH, CH=O, X, CH₂X, OR, OCOR, OPO(OR)₂, NHCOR wherein X is F, Cl, Br;
R₁ is H, CH₃, CH=O, CH₂OH;
R₂ is H, F OR Cl;
R₃ is H, CH₃ or X wherein X is F, Cl, Br;
R₄ is H, OH, CH₃, Alkyl, Ph, X, OCH₃, SH, NH₂, OR, SCOCH₃, SCOR, OCOR, CH₂OH, CH₂X, CH=O, CR=O, SR or NHCOR wherein X is F, Cl, Br;
R₅ is H, CH₃, CH₂CH₃, OH, SH, NH₂ or X wherein X is F, Cl, Br;
R₆ is H, OH, SH, NH2, CH₂CH₃, CH₃ or X wherein X is F, Cl, Br;
R₇ is H, OH, SH, NH₂, NO₂, CH=O, CH₃, CO₂H, CN, CH₂CH₃, CH₂X or X wherein X is F, Cl, Br; and
wherein said compound is not a compound disclosed in United States Patent 6,011,023 where the compound is to be used to treat or prevent ocular diseases selected from the list comprising retinal diseases (diabetic retinopathy, chronic glaucoma, retinal detachment, sickle cell retinopathy, senile macular degeneration due to subretinal neovascutarization), rubeosis iritis, inflammatory diseases, chronic uveitis, neoplasms (retinoblastoma, pseudoglioma), Fuchs' heterochromic iridocyclitis, neovascular glaucoma, corneal neovascularization (inflammatory, transplantation, developmental hypoplasia of the iris, corneal graft neovascularization), neovascularization following a combined vitrectomy and lensectomy, vascular diseases (retinal ischemia, choroidal vascular insufficiency, choroidal thrombosis, carotid artery ischemia), pterygium, neovascularization of the optic nerve, neovascularization due to penetration of the eye or contusive ocular injury, and retrolental fibroplasia.

Also provided by the description is a treatment of an ophthalmic condition in an individual comprising the step of: administering to the ophthalmic condition in the individual a therapeutically effective pharmaceutically acceptable formulation of a combination of:
(a) a mineralocorticoid wherein said mineralocorticoid is (i) capable of modulating the activity of mineralocorticoid receptors within cells or tissue located in an eye or adjacent to an eye to treat or prevent an ophthalmic condition; (ii) in a pharmaceutically acceptable form suitable for delivery to the eye; and
(b) at least a second compound in a concentration and dose sufficient to reduce ocular neovascularization wherein the second compound is selected from the group consisting of: a tetracycline derivative, a steroid, heparin, an antimicrobial, an anti-prostaglandin, and/or a metalloproteinase inhibitor.

A composition is also provided for the treatment of an ophthalmic condition in an individual comprising the step of: administering to the ophthalmic condition in the individual a therapeutically effective pharmaceutically acceptable formulation of a combination of:
(a) a mineralocorticoid wherein said mineralocorticoid is: (i) capable of modulating the activity of mineralocorticoid receptors within cells or tissue located in an eye or adjacent to an eye to treat or prevent an ophthalmic condition; and (ii) in a pharmaceutically acceptable form suitable for delivery to the eye; and
(b) a plurality of compounds in a concentration and dose sufficient to reduce ocular neovascularization wherein the plurality of compounds are selected from the group consisting of: a tetracycline derivative, a steroid, heparin, an antimicrobial, an anti-prostaglandin, and/or a metalloproteinase inhibitor.

In another aspect, the description provides a pharmaceutical composition for the treatment or prophylaxis of ophthalmic conditions comprising a therapeutically effective amount of a compound capable of modulating the activity of mineralocorticoid receptors within cells or tissues located in the eye. Preferably, said compound is an anti-oedematous steroid, more preferably a mineralocorticoid.

In a further aspect, the description provides for the use of a compound capable of modulating the activity of mineralocorticoid receptors within cells or tissue located in an eye or adjacent to an eye to treat or prevent an ophthalmic condition in the manufacture of a medicament for the treatment of an ophthalmic condition. Preferably, said compound is an anti-oedematous steroid, more preferably a mineralocorticoid.

The description also provides for the use of a compound capable of modulating the activity of mineralocorticoid receptors in the manufacture of a medicament for the treatment of an exudative retinopathy in an individual.

### Brief Description of the Drawings

Comprehension of the invention is facilitated by reading the following detailed description, in conjunction with the annexed drawings.
**Figure 1** shows the data from a fluorescence activated cell sorting (FACS) of choroidal endothelial cells comparing untreated cells against cells treated with phorbolmyristate acetate (PMA), cells treated with the glucocorticoid triamcinolone acetonide and cells treated with the mineralocorticoid fludrocortisone.
**Figure 2** shows the data from FACS on choroidal endothelial cells comparing untreated cells against cells treated with the mineralocorticoid fludrocortisone and cells treated with Tumour Necrosis Factor-α.
**Figure 3** shows the transendothelial electrical resistance (TER) of choroidal endothelial cells treated with dimethyl sulfoxide (DMSO), cells treated with the mineralocorticoid fludrocortisone, and cells treated with the glucocorticoid triamcinolone acetonide.
**Figure 4** shows the TER of retinal endothelial cells treated with DMSO, cells treated with the mineralocorticoid fludrocortisone, and cells treated with the glucocorticoid triamcinolone acetonide.
**Figure 5** shows a graph of the effect of the mineralocorticoids 11-desoxycortisone, deoxycorticosterone acetate, aldosterone, fludrocortisone acetate and deoxycorticosterone on bacterial endotoxin induced exudative
response in rabbits [C = control; 11-DC = 11-desoxycortisone; DA = deoxycorticosterone acetate; AS = aldosterone; FA = fludrocortisone acetate; DS= deoxycorticosterone]. (mean n = 5; Wilcoxon signed rank test)

### Description of the Invention

### General

The disclosure also includes all of the steps, features, formulations and compounds referred to or indicated in the specification, individually or collectively and any and all combinations or any two or more of the steps or features.

The invention described herein may include one or more range of values (eg size, concentration etc). A range of values will be understood to include all values within the range, including the values defining the range, and values adjacent to the range which lead to the same or substantially the same outcome as the values immediately adjacent to that value which defines the boundary to the range.

The file of this patent contains at least one drawing executed in colour. Copies of this patent with colour drawing(s) will be provided by the Patent and Trademark Office upon request and payment of the necessary fee.

Throughout this specification, unless the context requires otherwise, the word "comprise" or variations such as "comprises" or "comprising", will be understood to imply the inclusion of a stated integer or group of integers but not the exclusion of any other integer or group of integers. It is also noted that in this disclosure and particularly in the claims and/or paragraphs, terms such as "comprises, "comprised", "comprising" and the like can have the meaning attributed to it in U.S. Patent law; e.g., they can mean "includes", "included", "including", and the like; and that terms such as "consisting essentially of" and "consists essentially of" have the meaning ascribed to them in U.S. Patent law, e.g., they allow for elements not explicitly recited, but exclude elements that are found in the prior art or that affect a basic or novel characteristic of the invention.

Other definitions for selected terms used herein may be found within the description of the invention and apply throughout. Unless otherwise defined, all other scientific and technical terms used herein have the same meaning as commonly understood to one of ordinary skill in the art to which the invention belongs.

### Detailed Disclosure of the invention

This invention is based on the unexpected discovery that by contacting mineralocorticoid receptors within or adjacent to ocular tissue with agents that are capable of activating the mineralocorticoid receptor it is possible to ameliorate ophthalmic conditions in a patient's eye.

In one aspect of the present description, there is provided a treatment of an individual with an ophthalmic condition comprising the step of:
administering to the individual a therapeutically effective amount of a compound capable of modulating the activity of mineralocorticoid receptors within cells or tissue located in an eye or adjacent to an eye in the individual to be treated.

Preferably, the compound capable of modulating the activity of mineralocorticoid receptors within cells or tissue located in an eye or adjacent to an eye is an anti-oedematous steroid. More preferably, the anti-oedematous steroid is a mineralocorticoid. Even more preferably the compound is a compound that is not disclosed in United States Patent 6,011,023. If, however, the compound is a compound disclosed in United States Patent 6,011,023, the compound is not used to treat an ocular disease selected from the list comprising retinal diseases (diabetic retinopathy, chronic glaucoma, retinal detachment, sickle cell retinopathy, senile macular degeneration due to subretinal neovascularization), rubeosis iritis, inflammatory diseases, chronic uveitis, neoplasms (retinoblastoma, pseudoglioma), Fuchs' heterochromic iridocyclitis, neovascular glaucoma, corneal neovascularization (inflammatory, transplantation, developmental hypoplasia of the iris, corneal graft neovascularization), neovascularization following a combined vitrectomy and lensectomy, vascular diseases (retinal ischemia, choroidal vascular insufficiency, choroidal thrombosis, carotid artery ischemia), pterygium, neovascularization of the optic nerve, neovascularization due to penetration of the eye or contusive ocular injury, and retrolental fibroplasia.

As used herein the phrase "a compound capable of modulating the activity of mineralocorticoid receptors" refers to compounds capable of entering a cell to reach the cytosol or the nuclear membrane where the mineralocorticoid receptors are located, which are able to modulate the activity of the receptor. The activity of the receptor will be modulated when the activity of the receptor changes as a consequence of the compound interacting with the receptor (eg by activating the receptor). Preferably such compounds will have a lipophilic profile, which will facilitate their entry into the cell.

The role of mineralocorticoids lies in regulating the concentration of minerals, particularly sodium and potassium, in extracellular fluids. They bind to the mineralocorticoid receptor, triggering a series of events that lead to an increase in epithelial sodium channel activity and increased salt (particularly sodium) absorption. This in turn leads to raised blood pressure and increased fluid volume. The present description has now shown that administration of such compounds to the eve, and in particular vascular areas of the eye such as the retinal region, the conjunctiva, ciliary body and iris, produces anti-oedematous effects. Such effects are of particular value in the treatment of exudative diseases such as exudative retinopathies, or anterior uveitis. It has recently been shown that ocular tissues including retinal cells, retinal pigment epithelial cells, the epithelium of ciliary bodies, the iris, cornea and lens all express mineralocorticoid receptors.

Preferably the compounds used in the method of the present description are mineralocorticoids. Mineralocorticoids are able to bind to and activate the mineralocorticoid receptor. A range of steroids have mineralocorticoid activities, that is they can bind the mineralocorticoid receptor and activate it, leading to changes in the concentration of sodium and potassium in extracellular fluids via an increase in epithelial sodium channel activity, modulation of sodium/potassium ATPase activity and increased salt (particularly sodium) absorption. Some corticosteroids have both mineralocorticoid and glucocorticoid activity and the method of the present description uses the mineralocorticoid activity of such steroids.

The term "ophthalmic condition" includes both 'back-of-eye' diseases involving the retina, macular, fovea etc in the posterior region of the eye, and 'front-of-eye' diseases which predominantly involve the tissues at the front-of-eye, such as the cornea, iris, ciliary body, conjunctiva etc. In a preferred form of the description 'ophthalmic condition' will be understood to refer to a front-of-eye disease or back-of-eye disease caused by either direct or indirect modulation of the mineralocorticoid receptors in cells or tissue in or adjacent to the eye.

Examples of back-of-eye disease include macular oedema such as clinical macular oedema or angiographic cystoid macular oedema arising from various aetiologies such as diabetes, exudative macular degeneration and macular oedema arising from laser treatment of the retina, age-related macular degeneration, retinopathy of prematurity (also known as retrolental fibroplasia), retinal ischemia and choroidal neovascularization, retinal diseases (diabetic retinopathy, diabetic retinal oedema, retinal detachment, senile macular degeneration due to sub-retinal neovascularization, myopic retinopathy); inflammatory diseases; uveitis associated with neoplasms such as retinoblastoma or pseudoglioma; neovascularization following vitrectomy; vascular diseases (retinal ischemia, choroidal vascular insufficiency, choroidal thrombosis, retinopathies resulting from carotid artery ischemia); neovascularization of the optic nerve.

Examples of front-of-eye diseases include pterygium, corneal neovascularization (due to inflammation, transplantation, developmental hypoplasia of the iris, lensectomy), corneal diseases or opacifications with an exudative or inflammatory component, diffuse lamellar keratitis, neovascularization due to penetration of the eye or contusive ocular injury; rubeosis iritis; Fuchs' heterochromic iridocyclitis; chronic uveitis; anterior uveitis; inflammatory conditions resulting from surgeries such as LASIK, LASEK, refractive surgery, IOL implantation; irreversible corneal oedema as a complication of cataract surgery; oedema as a result of insult or trauma (physical, chemical, pharmacological, etc); inflammation; conjunctivitis (eg. persistent allergic, giant papillary, seasonal intermittent allergic, perennial allergic, toxic, conjunctivitis caused by infection by bacteria, viruses or Chlamydia); keratoconjunctivitis (vernal, atopic, Sicca); iridocyclitis; iritis; scleritis; episcleritis; infectious keratitis; superficial punctuate keratitis; keratoconus; posterior polymorphous dystrophy; Fuch's dystrophies (corneal and endothelial); aphakic and pseudophakic bullous keratopathy; corneal oedema; scleral disease; ocular cicatrcial pemphigoid; pars planitis; Posner Schlossman syndrome; Behçet's disease; Vogt-Koyanagi-Harada syndrome; hypersensitivity reactions; ocular surface disorders; conjunctival oedema; Toxoplasmosis chorioretinitis; inflammatory pseudotumor of the orbit; chemosis; conjunctival venous congestion; periorbital cellulitis; acute dacryocystitis; non-specific vasculitis; sarcoidosis; cytomegalovirus infection.

Preferably, the ophthalmic condition treated by the uses of the present description is chosen from the list comprising: diffuse lamellar keratitis, corneal diseases or opacifications with an exudative or inflammatory component, myopic retinopathies, macular oedema such as clinical macular oedema or angiographic cystoid macular oedema arising from various aetiologies such as diabetes, exudative macular degeneration and macular oedema arising from laser treatment of the retina, diabetic retinopathy, age-related macular degeneration, retinopathy of prematurity, retinal ischemia and choroidal neovascularization and like diseases of the retina; anterior uveitis; inflammatory conditions resulting from surgeries such as LASIK, LASEK, refractive surgery, IOL Implantation; irreversible corneal oedema as a complication of cataract surgery; oedema as a result of insult or trauma (physical, chemical, pharmacological, etc); inflammation; conjunctivitis (eg. persistent allergic, giant papillary, seasonal intermittent allergic, perennial allergic, toxic, conjunctivitis caused by infection by bacteria, viruses or Chlamydia); keratoconjunctivitis (vernal, atopic, Sicca); iridocyclitis; iritis; scleritis; episcleritis; infectious keratitis; superficial punctuate keratitis; keratoconus; posterior polymorphous dystrophy; Fuch's dystrophies (corneal and endothelial); aphakic and pseudophakic bullous keratopathy; corneal oedema; scleral disease; ocular cicatrcial pemphigoid; pars planitis; Posner Schlossman syndrome; Behçet's disease; Vogt-Koyanagi-Harada syndrome; hypersensitivity reactions; ocular surface disorders; conjunctival oedema; Toxoplasmosis chorioretinitis; inflammatory pseudotumor of the orbit; chemosis; conjunctival venous congestion; periorbital cellulitis; acute dacryocystitis; non-specific vasculitis; sarcoidosis; or Cytomegalovirus infection.

In one embodiment, the description provides a treatment of an ophthalmic condition in an individual comprising the step of: administering to the individual a therapeutically effective amount of a compound capable of modulating the activity of mineralocorticoid receptors within cells or tissue located in an eye or adjacent to an eye in the individual to be treated, wherein the ophthalmic condition is selected from the group comprising: myopic retinopathies, macular oedema such as clinical macular oedema or angiographic cystoid macular oedema arising from various aetiologies such as diabetes, exudative macular degeneration and macular oedema arising from laser treatment of the retina, diabetic retinopathy, age-related macular degeneration, retinopathy of prematurity, retinal ischemia and choroidal neovascularization and like diseases of the retina; anterior uveitis; inflammatory conditions resulting from surgeries such as LASIK, LASEK, refractive surgery, IOL implantation; irreversible corneal oedema as a complication of cataract surgery; oedema as a result of insult or trauma (physical, chemical, pharmacological, etc); inflammation; conjunctivitis (eg. persistent allergic, giant papillary, seasonal intermittent allergic, perennial allergic, toxic, conjunctivitis caused by infection by bacteria, viruses or Chlamydia); keratoconjunctivitis (vernal, atopic, Sicca); iridocyclitis; iritis; scleritis; episcleritis; infectious keratitis; superficial punctuate keratitis; keratoconus; posterior polymorphous dystrophy; Fuch's dystrophies (corneal and endothelial); aphakic and pseudophakic bullous keratopathy; corneal oedema; scleral disease; ocular cicatrcial pemphigoid; pars planitis; Posner Schlossman syndrome; Behçet's disease; Vogt-Koyanagi-Harada syndrome; hypersensitivity reactions; ocular surface disorders; conjunctival oedema; Toxoplasmosis chorioretinitis; inflammatory pseudotumor of the orbit; chemosis; conjunctival venous congestion; periorbital cellulitis; acute dacryocystitis; non-specific vasculitis; sarcoidosis; or Cytomegalovirus infection.

In a further aspect of the description there is provided a treatment of an ophthalmic condition in an individual comprising the steps of:
administering to an individual a therapeutically effective amount of a compound capable of modulating the activity of mineralocorticoid receptors within cells or tissue located in an eye or adjacent to an eye in the individual to be treated, wherein the ophthalmic condition is an exudative retinopathy. The present description also provides a method for the prophylactic or therapeutic treatment of exudative retinopathies comprising administering a therapeutically effective amount of a compound capable of modulating the activity of mineralocorticoid receptors within cells or tissue located in an eye or adjacent to an eye to treat or prevent an ophthalmic condition, to at least a ocular tissue selected from the group consisting of: retinal cells, retinal pigment epithelial cells, the epithelium of ciliary bodies, the iris, cornea and lens.

In another embodiment the description provides the treatment of an ailment resulting from the breakdown of the BRB in the posterior region of the retina, said method comprising the step of: administering to an individual a therapeutically effective amount of a compound capable of modulating the activity of mineralocorticoid receptors within cells or tissue located in an eye or adjacent to an eye to said ailment. Generally, ailments located in the posterior region of the retina arising from breakdown of the BRB will include myopic retinopathies, macular oedema such as clinical macular oedema or angiographic cystoid macular oedema arising from various aetiologies such as diabetes, macular oedema arising from laser treatment of the retina and exudative macular degeneration.

According to another embodiment the description provides the treatment or prophylaxis of exudative retinopathies in a patient requiring said treatment or prophylaxis, comprising administering by intravitreal injection to said patient an effective amount of a therapeutically effective amount of a compound capable of modulating the activity of mineralocorticoid receptors within cells or tissue located in an eye or adjacent to an eye that is preferably sparingly soluble in the vitreous.

When the ophthalmic condition to be treated is present in one eye, often the other eye will also present with the condition in the future. For example, 50% of cases where a wet-AMD type lesion occurs in one eye, a lesion occurs in the other eye within one year. Prophylactic administration of a compound capable of modulating the activity of mineralocorticoid receptors within cells or tissue located in an eye or adjacent to an eye to treat or prevent an ophthalmic condition such as an anti-oedematous steroid, or more preferably a mineralocorticoid, may be useful. Prophylactic administration may also be useful in preparation for laser treatment of the retina.

An 'individual' is a vertebrate, preferably mammal, more preferably a human. Mammals include, but are not limited to, humans, sport animals and pets, such as dogs and horses.

The terms 'treat', 'treatment' or 'treating' are used synonymously herein to describe the prevention, slowing, reversal or stopping of the exudative retinopathies to which the present description is directed.

The phrase "therapeutically effective amount" is used herein to refer to an amount of therapeutic agent either as an individual compound or in combination with other compounds that is sufficient to induce a therapeutic effect on the ailment which the compound is applied to. This phrase should not be understood to mean that the dose must completely eradicate the ailment. What constitutes a therapeutically effective amount will vary depending on, *inter alia,* the biopharmacological properties of the compound used in the methodology, the condition being treated, the frequency of administration, the mode of delivery, characteristics of the individual to be treated the severity of the disease and the response of the patient. These are the types of factors that a skilled pharmaceutical chemist will be aware of and will be able to account for when formulating compositions for a treatment as here in described.

An effective quantity of the compound of interest is preferably employed in the method of the description. For ocular and extraocular formulations, the concentration of the therapeutic compound may be in the range of about 0.01% w/w to about 10% w/w. Typically, the concentration for this mode of delivery is in the range of about 0.025% w/w to about 2.5% w/w.

In one aspect of the method of the description, the compound capable of modulating the activity of mineralocorticoid receptors within cells or tissue located in an eye or adjacent to an eye to treat or prevent an ophthalmic condition in accordance with the method of the description is a mineralocorticoid of the general structure: 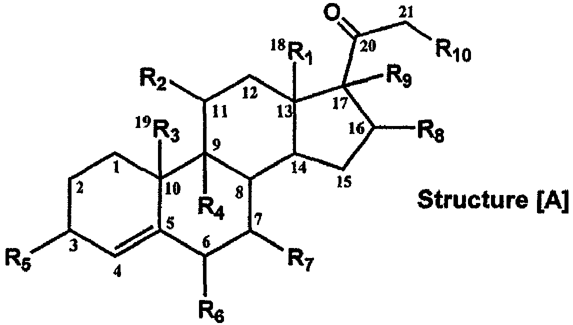 wherein R₁ is CH₃, CH=O, CH₂OH, CR=O, CH₂NH₂, COCHCH₂, NO₂ X or CH₂X wherein X is F, Cl, Br;
R₂ is H, OH, =O, NH₂ or CN;
R₃ is H, CH₃ or CH₂OH;
R₄ is H, F or Cl;
R₅ is H, OH, =O, SH, NH₂, CN, NO₂, CH₃, CH₂OH, CH=O, X, CH₂X, OR, OCOR, OPO(OR)₂, =CH₂ or CHR wherein X is F, Cl, Br;
R₆ is H, CH₃ or X wherein X is F, Cl, Br;
R₇ is H, OH, CH₃, Alkyl, Ph, X, OCH₃, OR, SCOCH₃, SCOR, OCOR, CH₂OH, CH₂X, CH=O, CR=O or NHCOR wherein X is F, Cl, Br;
R₈ is H, OH, CH₃, CH₂CH₃, SH, NH₂ or X wherein X is F, Cl, Br;
R₉ is H, OH, SH, NH₂, CH₃, CH₂CH₃ or X wherein X is F, Cl, Br;
R₁₀ is H, OH, CH₃, CH₂CH₃, SH, NH₂, CH₂OH, CH₂CH₂OH, OR, OCOR, OPO(OR)₂, NHCOR CH=O, X or CH₂X wherein X is F, Cl, Br; and
wherein said compound is not a compound disclosed in United States Patent 6,011,023 where the compound is to be used to treat or prevent ocular diseases selected from the list comprising retinal diseases (diabetic retinopathy, chronic glaucoma, retinal detachment, sickle cell retinopathy, senile macular degeneration due to subretinal neovascularization), rubeosis iritis, inflammatory diseases, chronic uveitis, neoplasms (retinoblastoma, pseudoglioma), Fuchs' heterochromic iridocyclitis, neovascular glaucoma, corneal neovascularization (inflammatory, transplantation, developmental hypoplasia of the iris, corneal graft neovascularization), neovascularization following a combined vitrectomy and lensectomy, vascular diseases (retinal ischemia, choroidal vascular insufficiency, choroidal thrombosis, carotid artery ischemia), pterygium, neovascularization of the optic nerve, neovascularization due to penetration of the eye or contusive ocular injury, and retrolental fibroplasia.

Preferably, the compound of Structure [A] is used in the uses of the present description to treat or prevent an ophthalmic condition selected from the list comprising: diffuse lamellar keratitis, corneal diseases or opacifications with an exudative or inflammatory component, myopic retinopathies, macular oedema such as clinical macular oedema or angiographic cystoid macular oedema arising from various aetiologies such as diabetes, exudative macular degeneration and macular oedema arising from laser treatment of the retina, diabetic retinopathy, age-related macular degeneration, retinopathy of prematurity, retinal ischemia and choroidal neovascularization and like diseases of the retina; anterior uveitis; inflammatory conditions resulting from surgeries such as LASIK, LASEK, refractive surgery, IOL implantation; irreversible corneal oedema as a complication of cataract surgery; oedema as a result of insult or trauma (physical, chemical, pharmacological, etc); inflammation; conjunctivitis (eg. persistent allergic, giant papillary, seasonal intermittent allergic, perennial allergic, toxic, conjunctivitis caused by infection by bacteria, viruses or Chlamydia); keratoconjunctivitis (vernal, atopic, Sicca); iridocyclitis; iritis; scleritis; episcleritis; infectious keratitis; superficial punctuate keratitis; keratoconus; posterior polymorphous dystrophy; Fuch's dystrophies (corneal and endothelial); aphakic and pseudophakic bullous keratopathy; corneal oedema; scleral disease; ocular cicatrcial pemphigoid; pars planitis; Posner Schlossman syndrome; Behçet's disease; Vogt-Koyanagi-Harada syndrome; hypersensitivity reactions; ocular surface disorders; conjunctival oedema; Toxoplasmosis chorioretinitis; inflammatory pseudotumor of the orbit; chemosis; conjunctival venous congestion; periorbital cellulitis; acute dacryocystitis; non-specific vasculitis; sarcoidosis; or cytomegalovirus infection.

In another aspect of the description, the compound capable of modulating the activity of mineralocorticoid receptors within cells or tissue located in an eye or adjacent to an eye to treat or prevent an ophthalmic condition in accordance with the method of the description is a mineralocorticoid of the general structure: 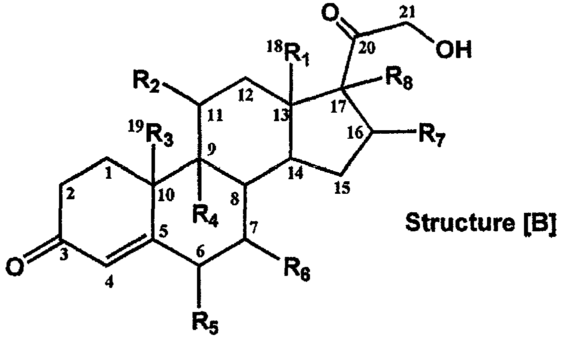 wherein
R₁ is CH₃, CH=O or COCHCH₂;
R₂ is H, OH or =O;
R₃ is H, CH₃ or CH₂OH;
R₄ is H, or F;
R₅ R₆ and R₇ are H; and
R₈ is H or OH; and
wherein said compound is not a compound disclosed in United States Patent 6,011,023 where the compound is to be used to treat or prevent ocular diseases selected from the list comprising retinal diseases (diabetic retinopathy, chronic glaucoma, retinal detachment, sickle cell retinopathy, senile macular degeneration due to subretinal neovascularization), rubeosis iritis, inflammatory diseases, chronic uveitis, neoplasms (retinoblastoma, pseudoglioma), Fuchs' heterochromic iridocyclitis, neovascular glaucoma, corneal neovascularization (inflammatory, transplantation, developmental hypoplasia of the iris, corneal graft neovascularization), neovascularization following a combined vitrectomy and lensectomy, vascular diseases (retinal ischemia, choroidal vascular insufficiency, choroidal thrombosis, carotid artery ischemia), pterygium, neovascularization of the optic nerve, neovascularization due to penetration of the eye or contusive ocular injury, and retrolental fibroplasia.

Preferably, the compound of Structure [B] is used in the uses of the present description to treat or prevent an ophthalmic condition selected from the list comprising: diffuse lamellar keratitis, corneal diseases or opacifications with an exudative or inflammatory component, myopic retinopathies, macular oedema such as clinical macular oedema or angiographic cystoid macular oedema arising from various aetiologies such as diabetes, exudative macular degeneration and macular oedema arising from laser treatment of the retina, diabetic retinopathy, age-related macular degeneration, retinopathy of prematurity, retinal ischemia and choroidal neovascularization and like diseases of the retina; anterior uveitis; inflammatory conditions resulting from surgeries such as LASIK, LASEK, refractive surgery, IOL implantation; irreversible corneal oedema as a complication of cataract surgery; oedema as a result of insult or trauma (physical, chemical, pharmacological, etc); inflammation; conjunctivitis (eg. persistent allergic, giant papillary, seasonal intermittent allergic, perennial allergic, toxic, conjunctivitis caused by infection by bacteria, viruses or Chlamydia); keratoconjunctivitis (vernal, atopic, Sicca); iridocyclitis; iritis; scleritis; episcleritis; infectious keratitis; superficial punctuate keratitis; keratoconus; posterior polymorphous dystrophy; Fuch's dystrophies (corneal and endothelial); aphakic and pseudophakic bullous keratopathy; corneal oedema; scleral disease; ocular cicatrcial pemphigoid; pars planitis; Posner Schlossman syndrome; Behçet's disease; Vogt-Koyanagi-Harada syndrome; hypersensitivity reactions; ocular surface disorders; conjunctival oedema; Toxoplasmosis chorioretinitis; inflammatory pseudotumor of the orbit; chemosis; conjunctival venous congestion; periorbital cellulitis; acute dacryocystitis; non-specific vasculitis; sarcoidosis; Cytomegalovirus infection.

In a further aspect of the description, the compound capable of modulating the activity of mineralocorticoid receptors within cells or tissue located in an eye or adjacent to an eye to treat or prevent an ophthalmic condition in accordance with the method of the description is a mineralocorticoid of the general structure: 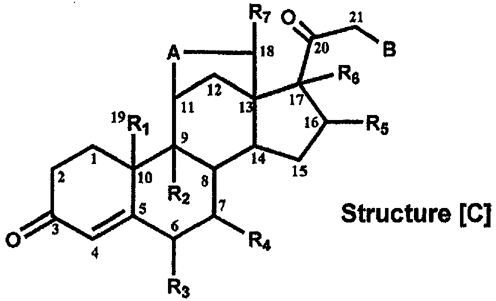 wherein
A is O, S, NH, CH₂, CHOH, C=O, CHX, CHCH₃, CH₂CH₂, OCH₂, SCH₂, NHCH₂, NRCH₂, NCOR wherein X is F, Cl, Br;
B is H, OH, SH, NH₂, CH₃, CH₂OH, CH₂CH₂OH, CH=O, X, CH₂X, OR, OCOR, OPO(OR)₂, NHCOR wherein X is F, Cl, Br;
R₁ is H, CH₃, CH=O, CH₂OH;
R₂ is H, F OR Cl;
R₃ is H, CH₃ or X wherein X is F, Cl, Br;
R₄ is H, OH, CH₃, Alkyl, Ph, X, OCH₃, SH, NH₂, OR, SCOCH₃, SCOR, OCOR, CH₂OH, CH₂X, CH=O, CR=O, SR or NHCOR wherein X is F, Cl, Br;
R₅ is H, CH₃, CH₂CH₃, OH, SH, NH₂ or X wherein X is F, Cl, Br;
R₆ is H, OH, SH, NH2, CH₂CH₃, CH₃ or X wherein X is F, Cl, Br; R₇ is H, OH, SH, NH₂, NO2, CH=O, CH₃, CO₂H, CN, CH₂CH₃, CH₂X or X wherein X is F, Cl, Br;
wherein said compound is not a compound disclosed in United States Patent 6,011,023 where the compound is to be used to treat or prevent ocular diseases selected from the list comprising retinal diseases (diabetic retinopathy, chronic glaucoma, retinal detachment, sickle cell retinopathy, senile macular degeneration due to subretinal neovascularization), rubeosis iritis, inflammatory diseases, chronic uveitis, neoplasms (retinoblastoma, pseudoglioma), Fuchs' heterochromic iridocyclitis, neovascular glaucoma, corneal neovascularization (inflammatory, transplantation, developmental hypoplasia of the iris, corneal graft neovascularization), neovascularization following a combined vitrectomy and lensectomy, vascular diseases (retinal ischemia, choroidal vascular insufficiency, choroidal thrombosis, carotid artery ischemia), pterygium, neovascularization of the optic nerve, neovascularization due to penetration of the eye or contusive ocular injury, and retrolental fibroplasia.

Preferably, the compound of Structure [C] is used in the uses of the present description to treat or prevent an ophthalmic condition selected from the list comprising: diffuse lamellar keratitis, corneal diseases or opacifications with an exudative or inflammatory component, myopic retinopathies, macular oedema such as clinical macular oedema or angiographic cystoid macular oedema arising from various aetiologies such as diabetes, exudative macular degeneration and macular oedema arising from laser treatment of the retina, diabetic retinopathy, age-related macular degeneration, retinopathy of prematurity, retinal ischemia and choroidal neovascularization and like diseases of the retina; anterior uveitis; inflammatory conditions resulting from surgeries such as LASIK, LASEK, refractive surgery, IOL implantation; irreversible corneal oedema as a complication of cataract surgery; oedema as a result of insult or trauma (physical, chemical, pharmacological, etc); inflammation; conjunctivitis (eg. persistent allergic, giant papillary, seasonal intermittent allergic, perennial allergic, toxic, conjunctivitis caused by infection by bacteria, viruses or Chlamydia); keratoconjunctivitis (vernal, atopic, Sicca); iridocyclitis; iritis; scleritis; episcleritis; infectious keratitis; superficial punctuate keratitis; keratoconus; posterior polymorphous dystrophy; Fuch's dystrophies (corneal and endothelial); aphakic and pseudophakic bullous keratopathy; corneal oedema; scleral disease; ocular cicatrcial pemphigoid; pars planitis; Posner Schlossman syndrome; Behçet's disease; Vogt-Koyanagi-Harada syndrome; hypersensitivity reactions; ocular surface disorders; conjunctival oedema; Toxoplasmosis chorioretinitis; inflammatory pseudotumor of the orbit; chemosis; conjunctival venous congestion; periorbital cellulitis; acute dacryocystitis; non-specific vasculitis; sarcoidosis; or cytomegalovirus infection.

The compound used in the method of the present description to treat or prevent an ocular condition by modulating the activity of mineralocorticoid receptors within cells or tissue located in an eye or adjacent to an eye may further be selected from the group comprising hydrocortisone hemisuccinate, hydrocortisone sodium phosphate, methylprednisolone hemisuccinate, prednisolone hemisuccinate, or prednisolone sodium succinate.

Alternatively, the compound capable of modulating the activity of mineralocorticoid receptors within cells or tissue located in an eye or adjacent to an eye to treat or prevent an ophthalmic condition in accordance with the method of the description may be selected from the group of compounds comprising:
dexamethasone sodium phosphate, hydrocortisone sodium succinate, methylprednisolone sodium succinate, wherein the ophthalmic condition to be treated or prevent is other than an ophthalmic condition selected from the list comprising: allergic conjunctivitis, keratitis, allergic corneal marginal ulcers, chorioretinitis, optic neuritis, anterior ischaemic optic neuropathy, iridocyclitis, iritis, diffuse posterior uveitis, choroiditis, sympathetic ophthalmia, anterior segment inflammation and herpes zoster ophthalmicus.

In a further alternative, the compound capable of modulating the activity of mineralocorticoid receptors within cells or tissue located in an eye or adjacent to an eye to treat or prevent an ophthalmic condition in accordance with the method of the description may be selected from the group of compounds comprising: deoxycorticosterone acetate, deoxycorticosterone pivalate, dexamethasone acetate, hydrocortisone butyrate, hydrocortisone valerate, prednisolone pivalate, prednisolone tebutate, wherein the ophthalmic condition to be treated or prevent is other than an ophthalmic condition selected from the list comprising retinal diseases (diabetic retinopathy, chronic glaucoma, retinal detachment, sickle cell retinopathy, senile macular degeneration due to subretinal neovascularization), rubeosis iritis, inflammatory diseases, chronic uveitis, neoplasms (retinoblastoma, pseudoglioma), Fuchs' heterochromic iridocyclitis, neovascular glaucoma, corneal neovascularization (inflammatory, transplantation, developmental hypoplasia of the iris, corneal graft neovascularization), neovascularization following a combined vitrectomy and lensectomy, vascular diseases (retinal ischemia, choroidal vascular insufficiency, choroidal thrombosis, carotid artery ischemia), pterygium, neovascularization of the optic nerve, neovascularization due to penetration of the eye or contusive ocular injury, and retrolental fibroplasia.

The compound capable of modulating the activity of mineralocorticoid receptors within cells or tissue located in an eye or adjacent to an eye to treat or prevent an ophthalmic condition in accordance with the method of the description may preferably be selected from the group of compounds comprising: cortisone, cortisone acetate, dexamethasone sodium phosphate, dexamethasone, hydrocortisone, hydrocortisone acetate, methylprednisolone, methylprednisolone acetate, prednisolone, prednisolone acetate, prednisolone sodium phosphate, prednisone, prednisone sodium phosphate, wherein the ophthalmic condition to be treated or prevented is other than an ophthalmic condition selected from the list comprising allergic conjunctivitis, keratitis, allergic corneal marginal ulcers, chorioretinitis, optic neuritis, anterior ischaemic optic neuropathy, iridocyclitis, iritis, diffuse posterior uveitis, choroiditis, sympathetic ophthalmia, anterior segment inflammation and herpes zoster ophthalmicus, nonpurulent conjunctivitis, blepharitis, scleritis, episcleritis, uveitis, retinal diseases (diabetic retinopathy, chronic glaucoma, retinal detachment, sickle cell retinopathy, senile macular degeneration due to subretinal neovascularization), rubeosis iritis, inflammatory diseases, chronic uveitis, neoplasms (retinoblastoma, pseudoglioma), Fuchs' heterochromic iridocyclitis, neovascular glaucoma, corneal neovascularization (inflammatory, transplantation, developmental hypoplasia of the iris, corneal graft neovascularization), neovascularization following a combined vitrectomy and lensectomy, vascular diseases (retinal ischemia, choroidal vascular insufficiency, choroidal thrombosis, carotid artery ischemia), pterygium, neovascularization of the optic nerve, neovascularization due to penetration of the eye or contusive ocular injury, and retrolental fibroplasia.

In another form of the description, the compound capable of modulating the activity of mineralocorticoid receptors within cells or tissue located in an eye or adjacent to an eye to treat or prevent an ophthalmic condition in accordance with the method of the description may be selected from the group of compounds comprising: amcinonide, clocortolone butyrate, flunisolide, flucinonide, flurandrenolide, fluticasone, fluticasone propionate, halincionide, mometasone furoate.

Alternatively, the compound capable of modulating the activity of mineralocorticoid receptors within cells or tissue located in an eye or adjacent to an eye to treat or prevent an ophthalmic condition in accordance with the method of the description may be selected from the group of compounds comprising:
clobetasol propionate, clobetasol butyrate, clocortolone butyrate, clobetasone pivalate, desoxymetasone, diflorasone, diflorasone diacetate, diflucortolone, diflucortolone valerate, flumethasone, flumethasone pivalate, fluocinolone, fluocortolone, fluocortolone hexanoate, fluocortolone pivalate, fluorometholone, isoflupredone, isoflupredone acetate, mometasone, wherein the ophthalmic condition to be treated or prevented is other than an ophthalmic condition selected from the list comprising retinal diseases (diabetic retinopathy, chronic glaucoma, retinal detachment, sickle cell retinopathy, senile macular degeneration due to subretinal neovascularization), rubeosis iritis, inflammatory diseases, chronic uveitis, neoplasms (retinoblastoma, pseudoglioma), Fuchs' heterochromic iridocyclitis, neovascular glaucoma, corneal neovascularization (inflammatory, transplantation, developmental hypoplasia of the iris, corneal graft neovascularization), neovascularization following a combined vitrectomy and lensectomy, vascular diseases (retinal ischemia, choroidal vascular insufficiency, choroidal thrombosis, carotid artery ischemia), pterygium, neovascularization of the optic nerve, neovascularization due to penetration of the eye or contusive ocular injury, and retrolental fibroplasia.

Rimexolone and fluocinolone acetonide may also be used in the method of the present description as the compound capable of modulating the activity of mineralocorticoid receptors within cells or tissue located in an eye or adjacent to an eye to treat or prevent an ophthalmic condition, wherein the ophthalmic condition to be treated or prevented is other than an ophthalmic condition selected from the list comprising: anterior chamber inflammation and anterior uveitis, retinal diseases (diabetic retinopathy, chronic glaucoma, retinal detachment, sickle cell retinopathy, senile macular degeneration due to subretinal neovascularization), rubeosis iritis, inflammatory diseases, chronic uveitis, neoplasms (retinoblastoma, pseudoglioma), Fuchs' heterochromic iridocyclitis, neovascular glaucoma, corneal neovascularization (inflammatory, transplantation, developmental hypoplasia of the iris, corneal graft neovascularization), neovascularization following a combined vitrectomy and lensectomy, vascular diseases (retinal ischemia, choroidal vascular insufficiency, choroidal thrombosis, carotid artery ischemia), pterygium, neovascularization of the optic nerve, neovascularization due to penetration of the eye or contusive ocular injury, and retrolental fibroplasia.

In a preferred form of the description, the compound capable of modulating the activity of mineralocorticoid receptors within cells or tissue located in an eye or adjacent to an eye to treat or prevent an ophthalmic condition in accordance with the method of the description is the mineralocorticoid aldosterone (11β-11,21-dihydroxy-3,20-dioxopregen-4-en-18-al; CAS 52-39-1) or an analogue, pharmaceutically acceptable salt, conjugate or derivative thereof that is preferably sparingly soluble in the vitreous.

Aldosterone may be isolated from living tissues by a number of methods such as those disclosed in Simpson et al (1953) Experimentia 9:333; Simpson et al (1954) Helv Chim Acta 37:1163; Mattox et al (1953) J Am Chem Soc 75:4869; or Harman et al (1954) J Am Chem Soc 76:5035.

Alternatively, aldosterone may be synthesised using methods derived from any one of the hollowing references: Schmidlin et al (1957) Helv Chim Acta 40:1483; Johnson et al (1958) J Am Chem Soc 80:2585; Johnson et al (1963) J Am Chem Soc 85:1409; Barton & Beaton (1960) J Am Chem Soc 82:2640; Barton & Beaton (1961) J Am Chem Soc 83:4083; Barton et al (1975) J Chem Soc Perkin Trans I 2243; or Miyano (1981) J Org Chem 46:1846.

In a highly preferred form of the description, the compound capable of modulating the activity of mineralocorticoid receptors within cells or tissue located in an eye or adjacent to an eye to treat or prevent an ophthalmic condition in accordance with the method of the description is a compound selected from the list comprising the mineralocorticoids: fludrocortisone (11β-9α-fluoro-11β,17α,21-trihydroxy-4-pregene-3,20-dione; CAS 127-31-1) or fludrocortisone acetate; deoxycorticosterone (cortexone or 21-hydroxypregn-4-ene-3,20-dione; CAS 64-85-7); 11-desoxycortisone (17α,21-dihydroxypregn-4-ene-3,20-dione; CAS 152-58-9); or deoxycorticosterone acetate (21-hydroxypregn-4-ene-3,20-dione 21 acetate; CAS 56-47-3) or an analogue, pharmaceutically acceptable salt, conjugate or derivative thereof that is preferably sparingly soluble in the vitreous, wherein the ophthalmic condition to be treated or prevent is other than an ophthalmic condition selected from the list comprising retinal diseases (diabetic retinopathy, chronic glaucoma, retinal detachment, sickle cell retinopathy, senile macular degeneration due to subretinal neovascularization), rubeosis iritis, inflammatory diseases, chronic uveitis, neoplasms (retinoblastoma, pseudoglioma), Fuchs' heterochromic iridocyclitis, neovascular glaucoma, corneal neovascularization (inflammatory, transplantation, developmental hypoplasia of the iris, corneal graft neovascularization), neovascularization following a combined vitrectomy and lensectomy, vascular diseases (retinal ischemia, choroidal vascular insufficiency, choroidal thrombosis, carotid artery ischemia), pterygium, neovascularization of the optic nerve, neovascularization due to penetration of the eye or contusive ocular injury, and retrolental fibroplasia.

Preferably, the fludrocortisone, fludrocortisone acetate, deoxycorticosterone, 11-desoxycortisone, or deoxycorticosterone acetate is used in the method of the present description to treat or prevent an ophthalmic condition selected from the list comprising: diffuse lamellar keratitis, corneal diseases or opacifications with an exudative or inflammatory component, myopic retinopathies, macular oedema such as clinical macular oedema or angiographic cystoid macular oedema arising from various aetiologies such as diabetes, exudative macular degeneration and macular oedema arising from laser treatment of the retina, diabetic retinopathy, age-related macular degeneration, retinopathy of prematurity, retinal ischemia and choroidal neovascularization and like diseases of the retina; anterior uveitis; inflammatory conditions resulting from surgeries such as LASIK, LASEK, refractive surgery, IOL implantation; irreversible corneal oedema as a complication of cataract surgery; oedema as a result of insult or trauma (physical, chemical, pharmacological, etc); inflammation; conjunctivitis (eg. persistent allergic, giant papillary, seasonal intermittent allergic, perennial allergic, toxic, conjunctivitis caused by infection by bacteria, viruses or Chlamydia); keratoconjunctivitis (vernal, atopic, Sicca); iridocyclitis; iritis; scleritis; episcleritis; infectious keratitis; superficial punctuate keratitis; keratoconus; posterior polymorphous dystrophy; Fuch's dystrophies (corneal and endothelial); aphakic and pseudophakic bullous keratopathy; corneal oedema; scleral disease; ocular cicatrcial pemphigoid; pars planitis; Posner Schlossman syndrome; Behçet's disease; Vogt-Koyanagi-Harada syndrome; hypersensitivity reactions; ocular surface disorders; conjunctival oedema; Toxoplasmosis chorioretinitis; inflammatory pseudotumor of the orbit; chemosis; conjunctival venous congestion; periorbital cellulitis; acute dacryocystitis; non-specific vasculitis; sarcoidosis; or cytomegalovirus infection.

Synthesis of fludrocortisone may be carried out using the method of Fried & Sabo (1954) J Am Chem Soc 76:1455, or alternatively fludrocortisone acetate may be synthesised using the method of US Patent 2,957,013.

Synthesis of deoxycorticosterone may be achieved using the method disclosed in Schindler et al (1941) Helv Chim Acta 24:371; DE Patent 875,353; DE Patent 871,153; US Patent 2,778,776; NL Patent 89,575; or DE Patent 1,028,572, and similar methods may be used to manufacture the related compound deoxycorticosterone acetate.

11-desoxycortisone synthesis may be carried out using the methods of any of the following articles: Reichstein & von Euw (1938) Helv Chim Acta 21:1197; Reichstein (1938) Helv Chim Acta 21:1490; Reichstein & von Euw (1940) Helv Chim Acta 23:1258; Sarett (1946) J Biol Chem 162:627; Gallagher et al (1949) J Am Chem Soc 71:3262; Julian et al (1949) J Am Chem Soc 71:3574; or Julian et al (1950) J Am Chem Soc 72:5145.

The preferred mineralocorticoids used in the uses of the present description may be administered alone, or in various combinations of two, three, four or more mineralocorticoids. For example, the mineralocorticoids may be administered in combinations of two mineralocorticoids such as:
aldosterone and fludrocortisone; aldosterone and deoxycorticosterone;
deoxycorticosterone and fludrocortisone acetate; deoxycorticosterone and 11-desoxycortisone; 11-desoxycortisone and fludrocortisone acetate aldosterone and
11-desoxycortisone; fludrocortisone and deoxycorticosterone; 11-desoxycortisone and fludrocortisone, aldosterone and deoxycorticosterone acetate, or any other combination.

Alternatively, the mineralocorticoids may be administered in triple combinations such as: aldosterone and fludrocortisone and 11-desoxycortisone; aldosterone and deoxycorticosterone and 11-desoxycortisone; fludrocortisone and deoxycorticosterone and 11-desoxycortisone; aldosterone and deoxycorticosterone and fludrocortisone acetate; or deoxycorticosterone acetate and 11-desoxycortisone and fludrocortisone or any other combination.

The mineralocorticoids may also be administered as a quadruple therapy if required eg. aldosterone, fludrocortisone, deoxycorticosterone and 11-desoxycortisone; or aldosterone, deoxycorticosterone acetate, 11-desoxycortisone and fludrocortisone acetate.

Combinations of five preferred compounds are also contemplated.

In another embodiment the description resides in the treatment of an ophthalmic condition in an individual comprising administering to the individual a therapeutically effective pharmaceutically acceptable formulation (that is, containing buffers and excipients as known to one skilled in the art) of a combination of: (a) a mineralocorticoid wherein said mineralocorticoid is (i) capable of modulating the activity of mineralocorticoid receptors within cells or tissue located in an eye or adjacent to an eye to treat or prevent an ophthalmic condition; and (ii) in a pharmaceutically acceptable form suitable for delivery to the eye; and (b) at least a second compound in a concentration and dose sufficient to reduce ocular neovascularization wherein the second compound is selected from the group consisting of: a tetracycline derivative, a steroid, heparin, an antimicrobial, an anti-prostaglandin, and/or a metalloproteinase inhibitor.

In one form, formulations used according to the description comprise a mineralocorticoid at a concentration from about 0.1 µg/ml to about 40 mg/ml and a steroid at a concentration from about 0.1 mg/ml to about 40 mg/ml.

Steroids are usually administered for ocular pathologies such as uveitis, diabetic retinopathy, idiopathic juxtafoveal telangiectasias, macular oedema secondary to diabetes mellitus, central retinal vein occlusion, pseudophakia, during photodynamic therapy for age related macular degeneration, etc., and for intraoperative visualization of the posterior hyaloid, which also desirably inhibits ocular neovascularization.

An undesirable and serious side effect of ocular steroid therapy is increased intraocular pressure, termed glaucoma or ocular hypertension. For patients with glaucoma or predisposed to glaucoma, steroid therapy presents a risk of unacceptably high intraocular pressure, such that surgery may be required to lower the intraocular pressure. Such risks and benefits must be balanced in determining whether to treat the patient with triamcinolone or other steroids.

The formulations and methods to predict patients at risk for glaucoma from steroid therapy, disclosed in co-pending U.S. Patent Application Serial No. 10/787,580, and PCT/AU2005/000263.

The co-pending applications provide a method to evaluate a patient's risk for the increased intraocular pressure that is known to occur in some patients receiving intraocular steroid therapy. In particular, a method is provided to evaluate a patient's risk for increased intraocular pressure after receiving intraocular steroid therapy comprising the steps of: (a) administering to an eye a steroid at a challenge dose ranging from about 50 µg to about 800 µg; and (b) determining what the intraocular pressure is in the eye after delivery of the challenge dose.

According to the method a steroid such as triamcinolone is administered at a challenge dose ranging from about 50 µg to about 800 µg, and the intraocular pressure is thereafter determined. In one embodiment, about 400 µg triamcinolone is administered. An intraocular pressure of at least 5 mm Hg higher after the challenge dose than an intraocular pressure before the challenge dose indicates that the patient would likely have increased intraocular pressure if a therapeutic dose of a steroid, such as about 4 mg to about 8 mg triamcinolone, were administered. The physician will then be better able to evaluate the benefits and risks of this therapy versus alternate therapy. The challenge dose may be injected into the vitreous of the eye, or it may be injected into another area or site in the eye, or it may be implanted in the eye, etc.

Steroids for ocular administration include, but are not limited to: triamcinolone (Aristocort®; Kenalog®), betamethasone (Celestone®), budesonide, fluorometholone (fluorometholone acetate (Flarex® (Alcon); Eflone®), fluorometholone alcohol (FML® and FML-Mild®, (Allergan); FluorOP®)), medrysone alcohol (HMSO (Allergan)); loteprednol etabonate (Lotemax® and Alrex® (Bausch & Lomb)), and anecortave acetate (Alcon). It will be appreciated that the above list is representative only and is not exclusive.

In a highly preferred form of the description the steroid used in the formulation is a 11-substituted-16α,17α-substituted methylenedioxy steroid selected from the compounds disclosed in United States Patent 5,770,589 to Billson and Penfold ("US '589"), which was filed as U.S. Application Serial No. 08/586,750. Alternatively, the compound is a steroid disclosed in Fried et al. (1958) J. Am. Chem. Soc. 80, 2338 (1958); U.S. Pat. No. 2,990,401; U.S. Pat. No. 3,048,581 or U.S. Pat. No. 3,035,050.

Collectively these publications also provide methods for the manufacture of such compounds and are also incorporated for the purposes of disclosing such methods. Desirably the steroid used is triamcinolone acetonide.

The steroid concentration in the formulation used in the method of the present description ranges from about 0.1 mg/ml to about 40 mg/ml. More preferably the steroid concentrations range from about 1 mg/ml to about 20 mg/ml. Alternatively, steroid concentrations range from about 20 mg/ml to about 30 mg/ml or they can range from about 20 mg/ml to about 40 mg/ml.

The steroid concentration used with a particular formulation will depend upon the particular steroid that is used. For example, triamcinolone acetonide (9α-fluoro-1 1 13, 16a, 17, 21 tetra hydroxy-pregna-1,4-diene-3,20-dione cyclic 16,17-acetal with acetone (C₂₄H₃₁F0₆)) Kenacort®, Kenalog® (Bristol-Myers Squibb, Princeton NJ) may be administered at a therapeutic dose in the range of about 4 mg to about 8 mg, for example, by intravitreous injection. In comparison, anecortave acetate, a steroid with less potential to cause an increase in intraocular pressure than triamcinolone but not used inside the eye, may be administered at a dose of about 0.5 mg/ml to about 30 mg/ml.

In a highly preferred form the following formulations may be used in the method of the present description: a 1:1 combination of about 5 µg/ml fludrocortisone and about 4 mg/ml triamcinolone acetonide; a 1:1 combination of about 5 µg/ml deoxycorticosterone acetate and about 4 mg/ml triamcinolone acetonide .

In a second form, formulations used in the method of the description comprise a mineralocorticoid in a concentration from about 0.1 µg/ml to about 40 mg/ml and heparin in a concentration from about 0.01 µg/ml to about 30 mg/ml.

Heparin is a heterogeneous group of straight-chain anionic mucopolysaccharides, termed glycosaminoglycans, having anticoagulant activity. The primary sugars are α-L-iduronic acid 2-sulfate, 2-deoxy-2-sulfamino-a-D-glucose 6-sulfate, (3-D-glucuronic acid, 2-acetamido-2-deoxy-α-D-glucose, and α-L- iduronic acid. These sugars are present in different amounts and are joined by glycosidic linkages, forming polymers of varying sizes. Heparin is strongly acidic because of its content of covalently linked sulfate and carboxylic acid groups. In heparin sodium, the acidic protons of the sulfates are partially replaced by sodium ions. In one embodiment of the description, low molecular weight heparin is used. Low molecular weight heparin is derived from unfractionated heparin through either chemical or enzymatic depolymerization, and is commercially available. Unfractionated heparin has a molecular weight of about 5,000 daltons to about 30,000 daltons, while low molecular weight heparin has a molecular weight of about 1,000 daltons to about 10,000 daltons. Compared to unfractionated heparin, low molecular weight heparin binds less strongly to protein, has enhanced bioavailability, interacts less with platelets and yields a predictable dose response and dose-dependent plasma levels, and produces less bleeding for a given antithrombotic effect. Low molecular weight heparin may be heparin sulfate, a lower-sulfated, higher-acetylated form of heparin. All of these are commercially available (e.g., Sigma Aldrich, St. Louis MO).

A possible mechanism for the beneficial effect of heparin or low molecular weight heparin in reducing vessel growth and proliferation is its polyanionic structure, which readily binds to polycationic angiogenic factors. Angiogenic factors with heparin bound to them have reduced biological activity, and therefore do not promote new vessel growth. *In vivo*, heparin sulfates are bound to the extracellular matrix (ECM) and endothelial cell surfaces. Heparin sulfate in the ECM may have a role in storing active growth factors that can be released when needed to exert immediate effects. Soluble heparins compete with heparin sulfates on the ECM for growth factors and proteins, and may consequently cause their release. Unfractionated heparin may cause an increase in the plasma level of growth factors. Unlike unfractionated heparin, which may promote angiogenesis, low molecular weight heparin may hinder the binding of growth factors to their high affinity receptors as a result of its smaller size. Low molecular weight heparin may affect the injured neovascular cornea by binding angiogenic factors that have been released from the ECM, as well as competitively (antagonistically) binding to angiogenic receptors.

In one embodiment, the concentration of heparin or low molecular weight heparin used in the method of the description to treat or prevent an ophthalmic conditions ranges from about 0.01 µg/ml to about 30 mg/ml. Alternatively, heparin or low molecular weight heparin may be administered in a concentration ranging from about 1 mg/ml to about 10 mg/ml. In a more preferred form of the description, the concentration of heparin or low molecular weight heparin ranges from about 0.5 mg/ml to about 15 mg/ml to 20 mg/ml (for example, administration of 0.1 ml of a 100 mg/ml formulation of low molecular weight heparin). In various embodiments, the concentration may be about 0.5 mg/ml to about 2.5 mg/ml, about 1 mg/ml to about 5 mg/ml, about 5 mg/ml to about 10 mg/ml, or about 5 mg/ml to about 30 mg/ml. Any concentration within these ranges may be used.

In a highly preferred form the following formulations may be used in the method of the present description: a 1:1 combination of about 5 µg/ml fludrocortisone and about 10 mg/ml low molecular weight heparin; a 1:1 combination of about 5 µg/ml deoxycorticosterone acetate and about 10 mg/ml low molecular weight heparin.

In a third form, formulations used in the method of the present description to treat or prevent an ophthalmic conditions comprise a mineralocorticoid in a concentration from about 0.1 µg/ml to about 40 mg/ml and an anti-prostaglandin in a concentration from about 1 µg/ml to about 10 mg/ml (such as a 1 µg/ml to about 10 mg/ml dose of flurbiprofen).

Anti-prostaglandins, also termed prostaglandin antagonists, may be administered in a concentration sufficient to result in a prostaglandin-inhibitory effect. As one example, antiprostaglandins such as flurbiprofen may be administered at a concentration in the range of about 0.001%^{w/v} to about 0.5%^{w/v}. As an example, OCUFEN® (flurbiprofen sodium 0.03% (Allergan), sodium (±)-2-(2-fluoro-4-biphenylyl)-propionate dihydrate) 0.03% may be administered at a concentration ranging from about 0.003%^{w/v} to about 0.3%^{w/v}. Anti-prostaglandins other than flurbiprofen may be included. The anti-prostaglandins may be administered at the doses and by the methods previously described, and include indomethacin, ketorolac, tromethamine 0.5% ((±)-5-benzoyl-2,3-dihydro-1H-pyrrolizine-1-carboxylic acid, compound with 2-amino-2-(hydroxymethyl)-1,3-propanediol (1:1) (ACULAR^{®} Allegan, Irvine CA), meclofenamate, fluorbiprofen, and compounds in the pyrrolo-pyrrole group of non-steroidal anti-inflammatory drugs (NSAIDs). For example, ACUALR® may be administered at a concentration ranging from about 0.003%^{w/v} to about 0.3%^{w/v}. In one embodiment, the concentration of ACULAR® is about 0.03%^{w/v}.

In specific embodiments, the following formulations may be used in the method of the present description: a 1:1 combination of about 0.03% ^{w/v} flurbiprofen and about 5 µg/ml fludrocortisone; a 1:1 combination of about 0.03% ^{w/v} flurbiprofen and about 5 µg/ml deoxycorticosterone acetate. For example, the formulation might comprise an actual concentration of 0.015% flurbiprofen with 5 µg/ml fludrocortisone or 0.015% flurbiprofen with 5 µg/ml deoxycorticosterone acetate.

In a fourth form, formulations used in the uses of the description comprise a mineralocorticoid in a concentration from about 0.1 µg/ml to about 40 mg/ml and an antimicrobial, like for example a macrolide antibiotic, in a concentration from about 20 µg/ml to about 200 µg/ml (about 0.002%^{w/v} to about 0.02%^{w/v}).

A possible mechanism for the beneficial effect of macrolide antibiotics are their anti-inflammatory effect.

Macrolide antibiotics that can be added to the formulation used in the treatment of ophthalmic conditions include, *inter alia*: tacrolimus, cyclosporine, sirolimus, everolimus, ascomycin, erythromycin, azithromycin, clarithromycin, clindamycin, lincomycin, dirithromycin, josamycin, spiramycin, diacetylmidecamycin, tylosin, roxithromycin, ABT-773, telithromycin, leucomycins, and lincosamide. Other antibiotics include, but are not limited to, aminoglycosides (e.g., streptomycin, amikacin, gentamicin, tobramycin), cephalosporins (e.g., beta lactams including penicillin), tetracyclines, acyclorvir, amantadine, polymyxin B, amphtotericin B, amoxicillin, ampicillin, atovaquone, azithromycin, azithromycin, bacitracin, cefazolin, cefepime, cefotaxime, cefotetan, cefpodoxime, ceftazidime, ceftizoxime, ceftriaxone, cefuroxime, cephalexin, chloramphenicol, clotimazole, ciprofloxacin, clarithromycin, clindamycin, dapsone, dicloxacillin, fluconazole, foscarnet, ganciclovir, gatifloxacin, griseofulvin, isoniazid, itraconazole, ketoconazole, metronidazole, nafcillin, neomycin, nitrofurantoin, nystatin, pentamidine, rifampin, rifamycin, valacyclovir, vancomycin, etc. The indications, effective doses, formulations, contraindications, vendors, etc. of these antibiotics are known to one skilled in the art.

Macrolide antibiotics can be administered in a concentration ranging from about 20 µg/ml to about 200 µg/ml (about 0.002%^{w/v} to about 0.02%^{w/v}). Formulations and doses of macrolide antibiotics are described in co-pending U.S. Patent Application Serial Nos. 10/667,161 and 10/752,124, each of which is expressly incorporated by reference herein in its entirety.

In addition to a macrolide antibiotic the formulation used can also include mycophenolic acid. Such a formulation when prepared as a pharmaceutically acceptable topically administered solution may include about 0.5%^{w/v} to about 10%^{w/v} mycophenolic acid. Preferably, a concentration of macrolide antibiotic and/or mycophenolic acid in a pharmaceutically acceptable topically administered solution may range from about 3%^{w/v} to about 5%^{w/v}. In another embodiment, a concentration of macrolide antibiotic and/or mycophenolic acid in a pharmaceutically acceptable topically administered solution may range from about 1 %^{w/v} to about 3%^{w/v}. In another embodiment, a concentration of macrolide antibiotic and/or mycophenolic acid in a pharmaceutically acceptable topically administered solution may range from about 3%^{w/v} to about 10%^{w/v}. In another embodiment, a concentration of macrolide antibiotic and/or mycophenolic acid may range from about 0.1 % to about 10% in a topical ocular formulation for treating diabetic retinopathy, age related macular degeneration, or retinitis pigmentosa. In another embodiment, concentrations of macrolide antibiotic and/or mycophenolic acid up to about 2%, up to about 5%, up to about 10%, or exceeding 10% are formulated for topical administration when the compound(s) is bound to a matrix or polymer which slowly releases the compound(s) over time while not exceeding an intraocular concentration of 40 µg/ml.

In a form of this embodiment, the description is treatment or prevention of an ophthalmic condition using a formulation comprising a mineralocorticoid in a concentration from about 0.1 µg/ml to about 40 mg/ml in a pharmaceutically acceptable form suitable for delivery to the eye and a tetracycline derivative (such as doxycycline, demeclocycline, minocycline, oxytetracycline, lymecycline, or a chemically modified tetracycline (CMT)) at a concentration from about 0.01 pg/ml to about 30 mg/ml.

As used herein a tetracycline or a derivative thereof, including CMTs which inhibit MMP activity, will include, *inter alia:* doxycycline, demeclocycline, minocycline, oxytetracycline, lymecydine, or a chemically modified tetracycline. Chemically modified tetracyclines (CMT) include demeclocycline, minocyciine, oxytetracycline and like compounds that inhibit the synthesis of MMP-8 and MMP-9. These include CMT such as CMT-315, CMT-3, CMT-8, and CMT-308; 6-demethyl-6-deoxy-4-dedimethylaminotetracylcine (COL-3), and others, for example, as described by Liu et al., A Chemically Modified Tetracycline (CMT-3) Is a New Antifungal Agent in Antimicrobial Agents Chemother. 2002 May; 46:1447; Seftor et al., Targeting the Tumor Microenvironment with Chemically Modified Tetracyclines: Inhibition of Laminin 5γ2 Chain Promigratory Fragments and Vasculogenic Mimicry 2002 November; 1: 1173, which are expressly incorporated by reference herein.

Tetracyclines exert their biological effects independent of their antimicrobial activity. That is, they inhibit MMPs and can prevent pathogenic tissue destruction. Furthermore, recent studies have suggested that tetracyclines and inhibitors of metalloproteinases suppress tumour progression, bone resorption, and angiogenesis and may have anti-inflammatory properties. Thus, a possible mechanism for the beneficial effect of tetracyclines and like compounds in reducing vessel growth and proliferation in the ocular region is via inhibition of metalloproteinases, which are zinc-dependent proteinase enzymes associated with the tumorigenic process. Selective inhibition of such metalloproteinase by the inventive formulations and methods described herein is believed to inhibit reactions leading to ocular neovascularization. Such metalloproteinase inhibitors are also included in the description.

In a highly preferred form of the description, the tetracycline derivative employed in the formulation is doxycycline. Doxycycline (4-(dimethylamino)-1,4,4a,5,5a,6,11,12a-octahydro-3,5,10,12,12a-pentahydroxy-6-methyl-1,11-dioxo-2-naphthacenecarboxamide monohydrate, C₂₂H₂₄N₂0₈.H₂0) is a commercially available broad spectrum antibiotic in the class of tetracycline antibiotics. The doxycycline in this form of the description is preferably present as a 2% solution of doxycycline at a substantially neutral pH in combination with the mineralocorticoid.

The concentration of doxycycline employed in this form of the description will range from 0.01 µg/ml to about 30 mg/ml. More specifically, doxycycline concentrations will range from about 0.05 mg/ml to about 1 mg/ml. Alternatively doxycycline concentrations will range from about 0.05 mg/ml to about 10 mg/ml. Yet again doxycycline concentrations can range from about 1 mg/ml to about 20 mg/ml. These doses are substantially non-toxic to the patient. Besides its anti-angiogenic effect, doxycycline could reduce the incidence of endophthalmitis, which occurs in about 0.5% of eyes in which a steroid is administered.

In a highly preferred form the formulation used in the uses of the description may comprise: a 1:1 combination of about 20 mg/ml doxycycline and about 5 µg/ml fludrocortisone; a 1:1 combination of about 20 mg/ml doxycycline and about 5 µg/ml deoxycorticosterone acetate.

In a fifth form, the formulation used in the present description comprises a mineralocorticoid in a concentration from about 0.1 µg/ml to about 40 mg/ml and an inhibitor of a metalloproteinase in a concentration and dose to reduce ocular neovascularization.

Inhibitors of metalloproteinases include naturally occurring proteins such as TIMP-1 that specifically inhibit matrix metalloproteinases, and synthetic metalloproteinase inhibitors such as Batimastat (BB-94) and marimastat (BB-2516) which potently and specifically inhibit metalloproteinase production. These inhibitors degrade the extracellular matrix, promoting tumor invasion and metastasis, but also regulate host defense mechanisms and normal cell function. Selective inhibition is expected to inhibit reactions leading to neovascularization in the inventive formulations and uses. Such metalloproteinase inhibitors are also included in the description. Among the twenty-four MMPs described, eight have been identified in the cornea, i.e., collagenase I and III (MMP-1 and MMP-13), gelatinase A and B (MMP-2 and -9), stromelysin (MMP-3), matrilysin (MMP-7) and membrane type MMP (MMP-14).

In an alternate embodiment of the description the formulation used to treat or prevent an ophthalmic conditions comprises: (a) a mineralocorticoid wherein said mineralocorticoid is: (i) capable of modulating the activity of mineralocorticoid receptors within cells or tissue located in an eye or adjacent to an eye to treat or prevent an ophthalmic condition; and (ii) in a pharmaceutically acceptable form suitable for delivery to the eye; and (b) a plurality of compounds in a concentration and dose to reduce ocular neovascularization, wherein the plurality of compounds are selected from the group consisting of: a tetracycline derivative, a steroid, heparin, an antimicrobial, an anti-prostaglandin, and/or a metalloproteinase inhibitor.

Where there are a plurality of tetracycline derivative, steroid, heparin, antimicrobial, anti-prostaglandin, and/or metalloproteinase inhibitor compounds employed in the method, the preferred compounds and their doses will be those which are described above.

In a further aspect of the present description, there is provideda treatment of an individual with an ophthalmic condition by administering to the individual a therapeutically effective amount of an anti-oedematous steroid or a pharmaceutically acceptable salt thereof; wherein said anti-oedematous steroid does not include compounds disclosed in United States Patent 6,011,023 when used to treat or prevent ocular diseases selected from the list comprising retinal diseases (diabetic retinopathy, chronic glaucoma, retinal detachment, sickle cell retinopathy, senile macular degeneration due to subretial neovascularization), rubeosis iritis, inflammatory diseases, chronic uveitis, neoplasms (retinoblastoma, pseudoglioma), Fuchs' heterochromic iridocyclitis, neovascular glaucoma, corneal neovascularization (inflammatory, transplantation, developmental hypoplasia of the iris, corneal graft neovascularization), neovascularization following a combined vitrectomy and lensectomy, vascular diseases (retinal ischemia, choroidal vascular insufficiency, choroidal thrombosis, carotid artery ischemia), pterygium, neovascularization of the optic nerve, neovascularization due to penetration of the eye or contusive ocular injury, and retrolental fibroplasia.

In one embodiment, the description provides treatment of an ophthalmic condition in an individual comprising: administering a dose of an anti-oedematous steroid or a pharmaceutically acceptable salt thereof, wherein the ophthalmic condition is selected from the group comprising: diffuse lamellar keratitis, corneal diseases or opacifications with an exudative or inflammatory component, myopic retinopathies, macular oedema such as clinical macular oedema or angiographic cystoid macular oedema arising from various aetiologies such as diabetes, exudative macular degeneration and macular oedema arising from laser treatment of the retina, diabetic retinopathy, age-related macular degeneration, retinopathy of prematurity, retinal ischemia and choroidal neovascularization and like diseases of the retina; anterior uveitis; inflammatory conditions resulting from surgeries such as LASIK, LASEK, refractive surgery, IOL implantation; irreversible corneal oedema as a complication of cataract surgery; oedema as a result of insult or trauma (physical, chemical, pharmacological, etc); inflammation; conjunctivitis (eg. persistent allergic, giant papillary, seasonal intermittent allergic, perennial allergic, toxic, conjunctivitis caused by infection by bacteria, viruses or Chlamydia); keratoconjunctivitis (vernal, atopic, Sicca); iridocyclitis; iritis; scleritis; episcleritis; infectious keratitis; superficial punctuate keratitis; keratoconus; posterior polymorphous dystrophy; Fuch's dystrophies (corneal and endothelial); aphakic and pseudophakic bullous keratopathy; corneal oedema; scleral disease; ocular cicatrcial pemphigoid; pars planitis; Posner Schlossman syndrome; Behçet's disease; Vogt-Koyanagi-Harada syndrome; hypersensitivity reactions; ocular surface disorders; conjunctival oedema; Toxoplasmosis chorioretinitis; inflammatory pseudotumor of the orbit; chemosis; conjunctival venous congestion; periorbital cellulitis; acute dacryocystitis; non-specific vasculitis; sarcoidosis; cytomegalovirus infection.

In a further aspect of the description, there is provided treatment of an ophthalmic condition in an individual comprising: administering a dose of an anti-oedematous steroid or a pharmaceutically acceptable salt thereof, wherein the ophthalmic condition is an exudative retinopathy. The present description also provides the prophylactic or therapeutic treatment of exudative retinopathies comprising administering a therapeutically effective amount of an anti-oedematous steroid, to at least an ocular tissue selected from the group consisting of: retinal cells, retinal pigment epithelial cells, the epithelial cells of the BRB, choroidal endothelial cells.

In another embodiment the description provides the treatment of an ailment resulting from the breakdown of the BRB in the posterior region of the retina, said method comprising the step of: administering a therapeutically effective amount of an anti-oedematous steroid to said ailment. Generally, ailments located in the posterior region of the retina arising from breakdown of the BRB will include myopic retinopathies, macular oedema such as clinical macular oedema or angiographic cystoid macular oedema arising from various aetiologies such as diabetes, macular oedema arising from laser treatment of the retina and exudative macular degeneration.

According to another embodiment the description provides the treatment or prophylaxis of exudative retinopathies in a patient requiring said treatment or prophylaxis, comprising administering by intravitreal injection to said patient an effective amount of anti-oedematous steroid or a pharmaceutically acceptable salt thereof that is preferably sparingly soluble in the vitreous.

Preferably, the anti-oedematous steroid used in the description is a mineralocorticoid steroid.

In a further aspect of the present description, there is provided treatment of an individual with an ophthalmic condition by administering to the individual a therapeutically effective amount of a mineralocorticoid or a pharmaceutically acceptable salt thereof as described herein; wherein said mineralocorticoid does not include compounds disclosed in United States Patent 6,011,023 when used to treat or prevent ocular diseases selected from the list comprising retinal diseases (diabetic retinopathy, chronic glaucoma, retinal detachment, sickle cell retinopathy, senile macular degeneration due to subretinal neovascularization), rubeosis iritis, inflammatory diseases, chronic uveitis, neoplasms (retinoblastoma, pseudoglioma), Fuchs' heterochromic iridocyclitis, neovascular glaucoma, corneal neovascularization (inflammatory, transplantation, developmental hypoplasia of the iris, corneal graft neovascularization), neovascularization following a combined vitrectomy and lensectomy, vascular diseases (retinal ischemia, choroidal vascular insufficiency, choroidal thrombosis, carotid artery ischemia), pterygium, neovascularization of the optic nerve, neovascularization due to penetration of the eye or contusive ocular injury, and retrolental fibroplasia.

In one embodiment, the description provides treatment of an ophthalmic condition in an individual comprising: administering a dose of a mineralocorticoid or a pharmaceutically acceptable salt thereof as described herein, wherein the ophthalmic condition is selected from the group comprising diffuse lamellar keratitis, corneal diseases or opacifications with an exudative or inflammatory component, myopic retinopathies, macular oedema such as clinical macular oedema or angiographic cystoid macular oedema arising from various aetiologies such as diabetes, exudative macular degeneration and macular oedema arising from laser treatment of the retina, diabetic retinopathy, age-related macular degeneration, retinopathy of prematurity, retinal ischemia and choroidal neovascularization and like diseases of the retina; anterior uveitis; inflammatory conditions resulting from surgeries such as LASIK, LASEK, refractive surgery, IOL implantation; irreversible corneal oedema as a complication of cataract surgery; oedema as a result of insult or trauma (physical, chemical, pharmacological, etc); inflammation; conjunctivitis (eg. persistent allergic, giant papillary, seasonal intermittent allergic, perennial allergic, toxic, conjunctivitis caused by infection by bacteria, viruses or Chlamydia); keratoconjunctivitis (vernal, atopic, Sicca); iridocyclitis; iritis; scleritis; episcleritis; infectious keratitis; superficial punctuate keratitis; keratoconus; posterior polymorphous dystrophy; Fuch's dystrophies (corneal and endothelial); aphakic and pseudophakic bullous keratopathy; corneal oedema; scleral disease; ocular cicatrcial pemphigoid; pars planitis; Posner Schlossman syndrome; Behçet's disease; Vogt-Koyanagi-Harada syndrome; hypersensitivity reactions; ocular surface disorders; conjunctival oedema; Toxoplasmosis chorioretinitis; inflammatory pseudotumor of the orbit; chemosis; conjunctival venous congestion; periorbital cellulitis; acute dacryocystitis; non-specific vasculitis; sarcoidosis; cytomegalovirus infection.

In a further aspect of the description, there is provided treatment of an ophthalmic condition in an individual comprising: administering a dose of a mineralocorticoid or a pharmaceutically acceptable salt thereof as described herein, wherein the ophthalmic condition is an exudative retinopathy.

The present description also provides a method for the prophylactic or therapeutic treatment of exudative retinopathies comprising administering a therapeutically effective amount of a mineralocorticoid, to at least an ocular tissue selected from the group consisting of: retinal cells, retinal pigment epithelial cells, the epithelial cells of the BRB, choroidal endothelial cells.

In another embodiment the description provides the treatment of an ailment resulting from the breakdown of the BRB in the posterior region of the retina, said method comprising the step of: administering a therapeutically effective amount of a mineralocorticoid as described herein to said ailment. Generally, ailments located in the posterior region of the retina arising from breakdown of the BRB will include myopic retinopathies, macular oedema such as clinical macular oedema or angiographic cystoid macular oedema arising from various aetiologies such as diabetes, macular oedema arising from laser treatment of the retina and exudative macular degeneration.

According to another embodiment the description provides the treatment or prophylaxis of exudative retinopathies in a patient requiring said treatment or prophylaxis, comprising administering by intravitreal injection to said patient an effective amount of mineralocorticoid or a pharmaceutically acceptable salt thereof that is preferably sparingly soluble in the vitreous.

The description also provides a pharmaceutical composition for the treatment or prophylaxis of ophthalmic conditions comprising a therapeutically effective amount of a compound capable of modulating the activity of mineralocorticoid receptors within cells or tissues located in the eye. Preferably such a compound is an anti-oedematous steroid.

The description further provides a pharmaceutical composition for the treatment or prophylaxis of ophthalmic conditions comprising a therapeutically effective amount of a mineralocorticoid as described herein. Preferably, the mineralocorticoid is aldosterone or a pharmaceutically acceptable analogue, salt, conjugate or derivative thereof together with a pharmaceutically acceptable additive. More preferably, the mineralocorticoid is selected from the group comprising fludrocortisone, fludrocortisone acetate; deoxycorticosterone, 11-desoxycortisone, or deoxycorticosterone acetate or a pharmaceutically acceptable thereof together with a pharmaceutically acceptable additive.

The description also provides a pharmaceutical composition for the treatment or prophylaxis of exudative retinopathies comprising a therapeutically effective amount of a mineralocorticoid selected from the group comprising fludrocortisone, fludrocortisone acetate; deoxycorticosterone, 11-desoxycortisone, or deoxycorticosterone acetate or a pharmaceutically acceptable thereof together with a pharmaceutically acceptable additive.

The description also provides a pharmaceutical composition for the treatment or prophylaxis of exudative retinopathies comprising a therapeutically effective amount of a mineralocorticoid or pharmaceutically acceptable salt thereof as described herein in a biocompatible, biodegradable matrix, for example in a topical form or in a form for intravitreal injection.

The description also provides a pharmaceutical composition or formulation for use in the methods of the description comprising a therapeutically effective amount of a compound capable of modulating the activity of mineralocorticoid receptors within cells or tissue located in an eye or adjacent to an eye (wherein said compound is preferably an anti-oedematous steroid or a pharmaceutically acceptable salt thereof and more preferably a mineralocorticoid) and at least a pharmaceutically acceptable additive (such as a carrier, adjunct, excipient or non-toxic, non-therapeutic, non-immunogenic stabilizers and the like). Preferably, the pharmaceutically acceptable additive should be compatible with the vitreous and should not leave any vision impairing residue in the eye. Desirably, any pharmaceutically acceptable additive used in the composition should be suited to the delivery of said pharmaceutical composition as an intravitreal depot injection.

The precise pharmaceutical formulation used in the present description will vary according to a wide range of commercial and scientific criteria. That is the skilled reader will appreciate that the above formulation of the description described above may contain other agents.

For example, the formulations used in the the description are preferably prepared using a physiological saline solution as a vehicle. The pH of the formulation may be maintained at a substantially neutral pH (for example, about 7.4, in the range of about 6.5 to about 7.4, etc.) with an appropriate buffer system as known to one skilled in the art (for example, acetate buffers, citrate buffers, phosphate buffers, borate buffers).

Any diluent used in the preparation of the pharmaceutically acceptable formulation may preferably be selected so as not to unduly affect the biological activity of the formulation. Examples of such diluents which are especially useful for injectable formulations are water, the various saline, organic or inorganic salt solutions, Ringer's solution, dextrose solution, and Hank's solution.

In addition, the pharmaceutical formulation used in the description may include additives such as other buffers, diluents, carriers, adjuvants or excipients. Any pharmacologically acceptable buffer suitable for application to the eye may be used, e.g., tris or phosphate buffers. Other agents may be employed in the formulation for a variety of purposes. For example, buffering agents, preservatives, co-solvents, surfactants, oils, humectants, emollients, chelating agents, stabilizers or antioxidants may be employed. Water soluble preservatives which may be employed include, but are not limited to, benzalkonium chloride, chlorobutanol, thimerosal, sodium bisulfate, phenylmercuric acetate, phenylmercuric nitrate, ethyl alcohol, methylparaben, polyvinyl alcohol, benzyl alcohol and phenylethyl alcohol. A surfactant may be Tween 80.

Other vehicles that may be used include, but are not limited to, polyvinyl alcohol, povidone, hydroxypropyl methyl cellulose, poloxamers, carboxymethyl cellulose, hydroxyethyl cellulose, purified water, etc. Tonicity adjustors may be included, for example, sodium chloride, potassium chloride, mannitol, glycerin, etc. Antioxidants include, but are not limited to, sodium metabisulfite, sodium thiosulfate, acetylcysteine, butylated hydroxyanisole, butylated hydroxytoluene, etc. The indications, effective doses, formulations, contraindications, vendors etc, of the compounds in the formulations are available or are known to one skilled in the art.

These agents may be present in individual amounts of from about 0.001% to about 5% by weight and preferably about 0.01% to about 2%. Suitable water soluble buffering agents that may be employed are sodium carbonate, sodium borate, sodium phosphate, sodium acetate, sodium bicarbonate, etc., as approved by the US FDA for the desired route of administration. These agents may be present in amounts sufficient to maintain a pH of the system of between about 2 to about 9 and preferably about 4 to about 8. As such, the buffering agent may be as much as about 5% (w/w) of the total formulation. Electrolytes such as, but not limited to, sodium chloride and potassium chloride may also be included in the formulation.

Compositions comprising the compound capable of modulating the activity of mineralocorticoid receptors within cells or tissue located in an eye or adjacent to an eye (which is preferably an anti-oedematous steroid or mineralocorticoid) used in the method of description are preferably composed of Ringer's lactate and optionally ethyl alcohol, preferably in an amount of 10-20 mg/ml of the total composition, more preferably 15 mg/ml of the total composition. Other solvents may be used, including water. Ethyl alcohol may have an adjuvant effect upon the compound used.

Solid dispersions of the therapeutic compound as well as solubilized preparations can be used to perform the treatment method of the description. For intraocular formulations (chemical delivery or delivery by invasive device), the therapeutic compound is delivered at a concentration high enough to achieve a final concentration in the range of about 0.1 µmol/L to about 10 µmol/L within the target ocular compartment (e.g. the posterior chamber for the treatment of retinal diseases). Typically, for this mode of delivery, the final concentration of the therapeutic compound is in the range of about 0.25 µmol/L to about 5 µmol/L.

In a highly preferred form of the description, when administering the mineralocorticoid by intravitreal injection for the prophylaxis or treatment of an ophthalmic condition, the mineralocorticoid should be as concentrated as feasible to minimise the volume for injection. The dosage of mineralocorticoid may be between about 1 µg and about 10 mg, or between about 1 mg and about 8 mg. Typically, about 4 mg of mineralocorticoid is deposited intravitreally. This dosage range is subject to the disease condition being treated.

The compositions of the present description for the treatment or prevention of ophthalmic conditions may be provided in the form of a single unit dose in a pre-prepared syringe, ready for administration.

For topical administration, the concentration of mineralocorticoid administered may depend upon the particular patient, the underlying disease and its severity, the dosing frequency, etc., as known to one skilled in the art. Sample concentrations include, but are not limited to, about 0.5 µg/ml to about 100 mg/ml, about 1 µg/ml to about 500 µg/ml, about 0.5 mg/ml to about 2.5 mg/ml, about 1 mg/ml to about 5 mg/ml, about 5 mg/ml to about 10 mg/ml, about 10 mg/ml to about 15 mg/ml, about 15 mg/ml up to 30 mg/ml, about 20 mg/ml to about 50 mg/ml, 30 mg/ml up to 100 mg/ml etc.

In performing the method of the description, pharmaceutically acceptable compounds may be administered to a patient by any method that leads to delivery of the therapeutic agent to the site of the ophthalmic condition (eg the location of an exudative retinopathy, anterior uveitis, pterygium, or macular oedema). Any of the formulations may be administered by an ocular route, such as topical, subconjunctival, sub-Tenon, intraocular, ocular implants etc.

Administration of the composition to perform the uses of the description is preferably by intraocular injection, although other modes of administration may be effective. Typically, pharmaceutically acceptable compounds will be delivered intraocularly (by chemical delivery system or invasive device) to an individual. However, the description is not limited to intraocular delivery in that it also includes topically (extraocular application) or systemically (e.g. oral or other parenteral route) provided that a sufficient amount of the compound capable of modulating the activity of mineralocorticoid receptors within cells or tissue located in an eye or adjacent an eye achieves contact with the site of the ophthalmic condition. Parenteral administration is used in appropriate circumstances apparent to the practitioner. Preferably, the compositions are administered in unit dosage forms suitable for single administration of precise dosage amounts.

As mentioned above, delivery to areas within the eye, *in situ* can be accomplished by injection, cannula or other invasive device designed to introduce precisely metered amounts of a desired formulation to a particular compartment or tissue within the eye (e.g. posterior chamber or retina). An intraocular injection may be into the vitreous (intravitreal), or under the conjunctiva (subconjunctival), or behind the eye (retrobulbar), into the sclera, or under the Capsule of Tenon (sub-Tenon), and may be in a depot form. Other intraocular routes of administration and injection sites and forms are also contemplated and are within the scope of the description.

Preferably, the intraocular injection is an intravitreal injection, preferably through self sealing 25-30 gauge needles or other suitably calibrated delivery device. Injection into the eye may be through the pars plana via the self-sealing needle. Most preferably, the compound capable of modulating the activity of mineralocorticoid receptors within cells or tissue located in an eye or adjacent an eye (preferably an anti-oedematous steroid or mineralocorticoid) is administered as an intravitreal depot injection.

In one embodiment, the formulation is intraocularly injected (eg, into the vitreous) to treat or prevent an ophthalmic condition. When administering the formulation by intravitreal injection, the active agents should be concentrated to minimise the volume for injection. Preferably, the volume for injection is less than about 5 ml. Volumes such as this may require compensatory drainage of the vitreous fluid to prevent increases in intraocular pressure and leakage of the injected fluid through the opening formed by the delivery needle. More preferably, the volume injected is between about 1.0 ml and 0.05 ml. Most preferably, the volume for injection is approximately 0.1 ml.

For injection, a concentration less than about 20 mg/ml may be injected, and any amount may be effective depending upon the factors previously described. Preferably a dose of less than 7 mg/ml is administered, with doses of less than 6 mg/ml, 5 mg/ml, 4 mg/ml 3 mg/ml, 2 mg/ml and 1 mg/ml being more preferred. Sample concentrations include, but are not limited to, about 5 µg/ml to about 50 µg/ml; about 25 µg/ml to about 100 µg/ml; about 100 µg/ml to about 200 µg/ml; about 200 µg/ml to about 500 µg/ml; about 500 µg/ml to about 750 µg/ml; about 500 µg/ml up to 1 mg/ml etc.

Intravitreal injection may be achieved by a variety of methods well known in the art. For example, the eye may be washed with a sterilising agent such as Betadine® and the mineralocorticoid is injected in an appropriate carrier with a fine gauge needle (eg 27 gauge) at a position in the eye such that the steroid crystals will settle to the posterior pole towards the ventral surface. It may be necessary to prepare the eye for injection by application of positive pressure prior to injection. In some cases, paracentesis may be necessary. Local anaesthetic or general anaesthetic may be necessary.

In one example, to prepare for injection topical alcaine was applied to the ocular surface, followed by 5% povidone iodine. A cotton-tipped applicator soaked in 4% lidocaine was then applied to the injection site, which is 4.0 mm posterior to the limbus in phakic eyes and 3.5 mm posterior to the limbus in pseudophakic eyes. A 27-gauge needle was used for injection at the superior pars plana. Indirect ophthalmoscopy can be used to confirm proper intravitreal placement of the suspension.

The syringe used in practicing the method of this description is suitably one which can accommodate a 21 to 30 gauge needle (eg a 23, 24, 25, 26 or 27 gauge needle) and is preferably of a small volume, for example 1.5 mL, or more preferably 0.5 mL. Although it is possible that the needle and syringe may be of the type where the needle is removable from the syringe, it is preferred that the arrangement is of a unitary syringe/needle construction. This would clearly limit the possibility of disengagement of the needle from the syringe. It is also preferred that the arrangement be tamper evident. The formulations of the present description may therefore be provided in the form of a single unit dose in a pre-prepared syringe, ready for administration.

A suitable style of syringe is, for example, sold under the name of Uniject^{™} manufactured by Becton Dickinson and Company. In this style of syringe, the material is expelled through the needle into the eye by pressure applied to the sides of a pliable reservoir supplying the needle, rather than by a plunger. As the name implies, the construction of the reservoir and needle forms a single unit.

Tropical application of formulations of the description for the treatment or prevention of ophthalmic conditions may be as ointment, gel or eye drops. Preferably a penetrating composition comprising the mineralocorticoid is used. The topical formulation may further be an *in situ* gellable aqueous formulation. Such a formulation comprises a gelling agent in a concentration effective to promote gelling upon contact with the eye or with lacrimal fluid in the exterior of the eye. Suitable gelling agents include, but are not limited to, thermosetting polymers such as tetra-substituted ethylene diamine block copolymers of ethylene oxide and propylene oxide (e.g., poloxamine); polycarbophil; and polysaccharides such as gellan, carrageenan (e.g., kappa-carrageenan and iota-carrageenan), chitosan and alginate gums.

The phrase *"in situ* gellable" as used herein embraces not only liquids of low viscosity that form gels upon contact with the eye or with lacrimal fluid in the exterior of the eye, but also more viscous liquids such as semi-fluid and thixotropic gels that exhibit substantially increased viscosity or gel stiffness upon administration to the eye. Indeed, it can be advantageous to formulate a formulation of the description as a gel, to minimize loss of the formulation immediately upon administration, as a result, for example, of lacrimation caused by reflex blinking. Although it is preferred that such a formulation exhibit further increase in viscosity or gel stiffness upon administration, this is not absolutely required if the initial gel is sufficiently resistant to dissipation by lacrimal drainage to provide the effective residence time specified herein.

To prepare a topical formulation for the treatment of ophthalmic conditions, a therapeutically effective amount of the formulation of the description is placed in an ophthalmological vehicle as is known in the art. For example, topical ophthalmic formulations containing steroids are disclosed in U.S. Pat. No. 5,041,434, whilst sustained release ophthalmic formulations of an ophthalmic drug and a high molecular weight polymer to form a highly viscous gel have been described in U.S. Pat. Nos. 4,271,143 and 4,407,792. Further, U.K. Patent Application GB 2007091 A, describes an ophthalmic composition in the form of a gel comprising an aqueous solution of a carboxyvinyl polymer, a water-soluble basic substance and an ophthalmic drug. Alternatively, U.S. Pat. No. 4,615,697, discloses a controlled release composition and method of use based on a bioadhesive and a treating agent, such as an anti-inflammatory agent.

The amount of the therapeutic compound to be administered and the concentration of the compound in the topical formulations used in the method depend upon the diluent, delivery system or device selected, the clinical condition of the patient, the side effects and the stability of the compound in the formulation. Thus, the physician employs the appropriate preparation containing the appropriate concentration of the therapeutic compound and selects the amount of formulation administered, depending upon clinical experience with the patient in question or with similar patients.

Where the formulation contains two or more active agents (eg two or more mineralocorticoids, or a mineralocorticoid and another agent such as a tetracycline derivative etc), the active agents may be administered as a mixture, as an admixture, in the same formulation, in separate formulations, in extended release formulations, liposomes, microcapsules, or any of the previously described embodiments. The formulation may be administered topically, or may be injected into the eye, or one active agent may be administered topically and the other agent(s) may be injected.

The formulation may be also administered as a slow release formulation, with a carrier formulation such as microspheres, microcapsules, liposomes, etc., as a topical ointment or solution, an intravenous solution or suspension, or in an intraocular injection, as known to one skilled in the art to treat or prevent an ophthalmic condition.

A time-release drug delivery system may be administered intraocularly to result in sustained release of the agent over a period of time. The formulation may be in the form of a vehicle, such as a micro- or macro-capsule or matrix of biocompatible polymers such as polycaprolactone, polyglycolic acid, polylactic acid, polyanhydrides, polylactide-co-glycolides, polyamino acids, polyethylene oxide, acrylic terminated polyethylene oxide, polyamides, polyethylenes, polyacrylonitriles, polyphosphazenes, poly(ortho esters), sucrose acetate isobutyrate (SAIB), and other polymers such as those disclosed in U.S. Patent Nos. 6,667,371; 6,613,355; 6,596,296; 6,413,536; 5,968,543; 4,079,038; 4,093,709; 4,131,648; 4,138,344; 4,180,646; 4,304,767; 4,946,931, each of which is expressly incorporated by reference herein in its entirety, or lipids that may be formulated as microspheres or liposomes. A microscopic or macroscopic formulation may be administered through a needle, or may be implanted by suturing within the eye, for example, within the lens capsule. Delayed or extended release properties may be provided through various formulations of the vehicle (coated or uncoated microsphere, coated or uncoated capsule, lipid or polymer components, unilamellar or multilamellar structure, and combinations of the above, etc.). The formulation and loading of microspheres, microcapsules, liposomes, etc. and their ocular implantation are standard techniques known by one skilled in the art, for example, the use a ganciclovir sustained-release implant to treat cytomegalovirus retinitis, disclosed in Vitreoretinal Surgical Techniques, Peyman et al., Eds. (Martin Dunitz, London 2001, chapter 45); Handbook of Pharmaceutical Controlled Release Technology, Wise, Ed. (Marcel Dekker, New York 2000), the relevant sections of which are incorporated by reference herein in their entirety. For example, a sustained release intraocular implant may be inserted through the pars plana for implantation in the vitreous cavity.

The description also provides the treatment or prophylaxis of ophthalmic condition such as exudative retinopathies comprising: administering a pharmaceutical composition comprising a therapeutically effective amount of a compound capable of modulating the activity of mineralocorticoid receptors within cells or tissue located in an eye or adjacent to an eye (which is preferably an anti-oedematous steroid or a pharmaceutically acceptable salt thereof, or more preferably a mineralocorticoid) in a biocompatible, biodegradable matrix, for example in the form of a gel or polymer which is preferably suited for insertion into the retina or into a cavity of the eye, anterior or posterior, as an implant. In the case that the composition is delivered as an implant, it may be incorporated in any known biocompatible biodegradable matrix as a liquid, or in the form, for example, of a micelle using known chemistry or as microparticles.

Slow or extended-release delivery systems include any of a number of biopolymers (biological-based systems), systems employing liposomes, colloids, resins, and other polymeric delivery systems or compartmentalized reservoirs, can be utilized with the compositions described herein to provide a continuous or long term source of therapeutic compound.

In any slow release device prepared, the mineralocorticoid receptor activity modulating compound is preferably present in an amount of about 10% to 90% by weight of the implant. More preferably, the compound is from about 50% to about 80% by weight of the implant. In a preferred embodiment, the mineralocorticoid receptor activity modulating compound comprises about 50% by weight of the implant. In a particularly preferred embodiment, the compound mineralocorticoid receptor activity modulating compound comprises about 70% by weight of the implant. In a preferred aspect, the mineralocorticoid receptor activity modulating compound is an anti-oedematous steroid or pharmaceutically acceptable salt thereof, more preferably a mineralocorticoid.

In one form, implants used in the method of the present description are formulated with particles of the mineralocorticoid entrapped within the bio-erodible polymer matrix. Release of the agent is achieved by erosion of the polymer followed by exposure of previously entrapped agent particles to the vitreous, and subsequent dissolution and release of agent. The release kinetics achieved by this form of drug release are different than that achieved through formulations which release drug through polymer swelling, such as with hydrogels such as methylcellulose. In that case, the drug is not released through polymer erosion, but through polymer swelling, which releases drug as liquid diffuses through the pathways exposed. The parameters which determine the release kinetics include the size of the drug particles, the water solubility of the drug, the ratio of drug to polymer, the method of manufacture, the surface area exposed, and the erosion rate of the polymer.

Exemplary biocompatible, non-biodegradable polymers of particular interest include polycarbamates or polyureas, particularly polyurethanes, polymers which may be cross-linked to produce non-biodegradable polymers such as cross-linked poly(vinyl acetate) and the like. Also of particular interest are ethylene-vinyl ester copolymers having an ester content of 4% to 80% such as ethylene-vinyl acetate (EVA) copolymer, ethylene-vinyl hexanoate copolymer, ethylene-vinyl propionate copolymer, ethylene-vinyl butyrate copolymer, ethylene-vinyl pentantoate copolymer, ethylene-vinyl trimethyl acetate copolymer, ethylene-vinyl diethyl acetate copolymer, ethylene-vinyl 3-methyl butanoate copolymer, ethylene-vinyl 3-3-dimethyl butanoate copolymer, and ethylene-vinyl benzoate copolymer.

Additional exemplary naturally occurring or synthetic non-biodegradable polymeric materials include poly(methylmethacrylate), poly(butylmethacrylate), plasticized poly(vinylchloride), plasticized poly(amides), plasticized nylon, plasticized soft nylon, plasticized poly(ethylene terephthalate), natural rubber, silicone, poly(isoprene), poly(isobutylene), poly(butadiene), poly(ethylene), poly(tetrafluoroethylene), poly(vinylidene chloride), poly(acrylonitrile, cross-linked poly(vinylpyrrolidone), poly(trifluorochloroethylene), chlorinated poly(ethylene), poly(4,4'-isopropylidene diphenylene carbonate), vinylidene chloride-acrylonitrile copolymer, vinyl chloridediethyl fumarate copolymer, silicone, silicone rubbers (especially the medical grade), poly(dimethylsiloxanes), ethylene-propylene rubber, silicone-carbonate copolymers, vinylidene chloride-vinyl chloride copolymer, vinyl chloride-acrylonitrile copolymer, vinylidene chloride-acrylonitrile copolymer, poly(olefins), poly(vinyl-olefins), poly(styrene), poly(halo-olefins), poly(vinyls), poly(acrylate), poly(methacrylate), poly(oxides), poly(esters), poly(amides), and poly(carbonates)

Diffusion of the drug from the implant may also be controlled by the structure of the implant. For example, diffusion of the drug from the implant may be controlled by means of a membrane affixed to the polymer layer comprising the drug. The membrane layer will be positioned intermediate to the polymer layer comprising the drug and the desired site of therapy. The membrane may be composed of any of the biocompatible materials indicated above and may vary with the drug employed, the presence of agents in addition to the drug present in the polymer, the composition of the polymer comprising the drug, the desired rate of diffusion and the like. For example, the polymer layer will usually comprise a very large amount of drug and will typically be saturated. Such drug-saturated polymers may generally release the drug at a very high rate. In this situation, the release of the drug may be slowed by selecting a membrane which is of a lower drug permeability than the polymer. Due to the lower drug permeability of the membrane, the drug will remain concentrated in the polymer and the overall rate of diffusion will be determined by the drug permeability of the membrane. Therefore, the rate of release of the drug from the implant is reduced, providing for a more controlled and extended delivery of the drug to the site of therapy.

The skilled reader will appreciate that the duration over which any of the formulations used in the method of the description will dwell in the ocular environment will depend, *inter alia,* on such factors as the physicochemical and/or pharmacological properties of the compounds employed in the formulation, the concentration of the compound employed, the bioavailability of the compound, the disease to be treated, the mode of administration and the preferred longevity of the treatment. Where that balance is struck will often depend on the longevity of the effect required in the eye and the ailment being treated.

The frequency of treatment according to the method of the description is determined according to the disease being treated, the deliverable concentration of the mineralocorticoid and the method of delivery. If delivering the mineralocorticoid by intravitreal injection, the dosage frequency may be monthly. Preferably, the dosage frequency is every three months. The frequency of dosage may also be determined by observation, with the dosage being delivered when the previously delivered mineralocorticoid material is visibly degraded, however one should be careful with such a measurement as the mineralocorticoid material may be visibly degraded, but may exist in dissolved therapeutic levels in the eye. Once a therapeutic result is achieved, the drug can be tapered or discontinued. Occasionally, side effects warrant discontinuation of therapy. In general, an effective amount of the compound is that which provides either subjective relief of symptoms or an objectively identifiable improvement as noted by the clinician or other qualified observer.

Formulations prepared for used in the method of the present description to prevent or treat ophthalmic conditions will preferably have dwell times from hours to many months and possibly years, although the latter time period requires special delivery systems to attain such a duration. Illustrative forms of such delivery systems are disclosed elsewhere in this specification (eg below).

Most preferably the formulations for use in the method of the description will have a dwell time (ie duration in the eye) of hours (i.e. 1 to 24 hours), days (i.e. 1, 2, 3, 4, 5, 6 or 7 days) or weeks (i.e. 1, 2, 3, 4 weeks). Alternatively, the formulation will have a dwell time of at least a few months such as, 1 month, 2 months, 3 months, with dwell times of greater than 4, 5, 6, 7 to 12 months being achievable.

Compounds capable of modulating the activity of mineralocorticoid receptors within cells or tissue located in an eye or adjacent to an eye to treat or prevent an ophthalmic condition (such as an anti-oedematous steroid, or more preferably a mineralocorticoid) suitable for use in the present description must be capable of at least reducing, ameliorating or preventing the exudation of fluids from the blood vessels of the eye (eg from the choroidal and retinal vasculature into the retinal space or from the vessels of the conjunctiva, iris or ciliary body into the front-of-eye region). Where a prolonged treatment is required, the mineralocorticoid receptor activity modulating compounds (eg anti-oedematous steroids or mineralocorticoids) are preferably formed of crystals that are sparingly soluble in the vitreous. Such compounds prepared in a crystalline manner may be administered in depot form.

Where the buffering agent or enhancer is hydrophilic, it may also act as a release accelerator. Hydrophilic additives act to increase the release rates through faster dissolution of the material surrounding the drug particles, which increases the surface area of the drug exposed, thereby increasing the rate of drug bioerosion. Similarly, a hydrophobic buffering agent or enhancer dissolves more slowly, slowing the exposure of drug particles, and thereby slowing the rate of drug bioerosion.

In order to provide slow release compositions with improved therapeutically effective dwell time in the vitreous for the method of treating or preventing ophthalmic conditions, the proportion of different crystal sizes of the mineralocorticoid receptor activity modulating compounds (eg anti-oedematous steroids, or more preferably mineralocorticoids) may be manipulated to provide a suitable release profile. A key to increasing the therapeutically effective intravitreal dwell time of an anti-oedematous steroid, such as, for example, fludrocortisone, without increasing the total concentration of the drug in a therapeutic formulation is to increase the proportion of larger-sized crystals of the anti-oedematous steroid while decreasing the proportion of crystal composites of the anti-oedematous steroid. Accordingly, the dissolution rate of an anti-oedematous steroid drug, such as, for example, fludrocortisone, may be decreased (i.e. longer dissolution time) by increasing the relative proportion of large-sized drug crystals in a therapeutic composition or formulation. The larger-sized drug crystals have a decreased surface area to volume relationship relative to crystal composites, and as such, have a lower rate of dissolution.

For example, the compounds may be present in the composition with a greater proportion of crystals of about 50 µm to about 600 µm than the proportion of crystals of about 0.5 µm to about 40 µm. As detailed in PCT/AU2005/000146 and US Patent Application 11/051,028 such compositions provide slow release compositions whose time release profile may be adjusted by altering the proportions of larger crystals to smaller crystals. To this end the compositions used in the present description may comprise a pharmaceutically acceptable composition comprising an anti-oedematous steroid or pharmaceutically acceptable salt thereof which is present in the form of crystals and crystal composites of varying sizes and wherein said crystals are concentrated in the size ranges of about 0.5 µm to about 40 µm and about 50 µm to about 600 µm. Preferably the crystals are more concentrated than the crystal composites in the size ranges of about 0.5 µm to about 40 µm and about 50 µm to about 600 µm. Even more preferably the proportion of crystals in the size ranges of about 50 µm to about 600 µm is greater than that provided in the about 0.5 µm to about 40 µm size range.

In another embodiment, the description resides in modulating the activity of mineralocorticoid receptors within cells or tissue located in an eye or adjacent to an eye to treat or prevent an ophthalmic condition comprising the step of: administering to a patient a compound capable of modulating the activity of mineralocorticoid receptors within cells or tissue located in an eye or adjacent to an eye in a pharmaceutically acceptable formulation suitable for delivery to the eye in an amount and for a duration sufficient to modulate the activity of mineralocorticoid receptors to treat or prevent an ophthalmic condition. It is preferred that the compound capable of modulating the activity of the mineralocorticoid receptors is an anti-oedematous steroid, most preferably a mineralocorticoid as defined herein.

Desirably, the use for modulating the activity of mineralocorticoid receptors consists of administering to a patient a formulation that contains at least aldosterone in a pharmaceutically acceptable formulation suitable for delivery to the eye in an amount and for a duration sufficient to treat or prevent an ophthalmic condition. More desirably the method for modulating the activity of mineralocorticoid receptors consists of administering to a patient a formulation that contains at least one of fludrocortisone, fludrocortisone acetate; deoxycorticosterone, 11-desoxycortisone, or deoxycorticosterone acetate in a pharmaceutically acceptable formulation suitable for delivery to the eye in an amount and for a duration sufficient to treat or prevent an ophthalmic condition wherein the ophthalmic condition to be treated or prevent is other than an ocular disease selected from the list comprising retinal diseases (diabetic retinopathy, chronic glaucoma, retinal detachment, sickle cell retinopathy, senile macular degeneration due to subretinal neovascularization), rubeosis iritis, inflammatory diseases, chronic uveitis, neoplasms (retinoblastoma, pseudoglioma), Fuchs' heterochromic iridocyclitis, neovascular glaucoma, corneal neovascularization (inflammatory, transplantation, developmental hypoplasia of the iris, corneal graft neovascularization), neovascularization following a combined vitrectomy and lensectomy, vascular diseases (retinal ischemia, choroidal vascular insufficiency, choroidal thrombosis, carotid artery ischemia), pterygium, neovascularization of the optic nerve, neovascularization due to penetration of the eye or contusive ocular injury, and retrolental fibroplasia.

In a more preferred form of the description the formulation used in the above method is a formulation described above to treat or prevent an ophthalmic condition in the anterior and/or posterior regions of the eye, or in the cornea, retina, choroid, etc.

The methods of treatment or prophylaxis of ophthalmic conditions of the present description may be performed alone, or in combination with one or more other therapies such as photodynamic therapy, laser surgery, laser photocoagulation or one or more biological or pharmaceutical treatments.

Laser treatment takes a number of forms, depending on the nature of the ophthalmic disorder. Disorders such as myopia may be treated with laser surgery to reshape the cornea (eg. LASIK® surgery), whilst a widely used treatment for disorders such as AMD is laser therapy which is directed to removal or blockage of blood vessels via photodynamic therapy or laser photocoagulation. Laser therapy may further be used to treat or remove neoplasm such as retinoblastomas or pseudogliomas.

Photocoagulation involves the use of a laser to seal leaking blood vessels, slow the growth of abnormal blood vessels and/or destroy new blood vessels within the eye. In addition, the laser can be used to seal the retina to the eye, helping to prevent retinal detachment. For example, focal laser treatment may be applied to microaneurysms identified in diabetic retinopathy.

Photodynamic therapy involves the use of a photoactive drug (eg Visudyne^{®}) and a laser to destroy abnormal blood vessels. Visudyne^{®} is injected into the blood and activated with a laser, effectively destroying the blood vessels. This treatment may require several sessions to be effective. A wide range of theories have been proposed to explain the beneficial effects of retinal laser photocoagulation in delaying retinal angiogenesis, however, the underlying molecular mechanism remains to be elucidated.

The therapeutic effects of laser photocoagulation are thought to be due to the destruction of photoreceptors, the highest oxygen consumers in the retina. Subsequently, these photoreceptors are replaced by glial cells allowing increased oxygen diffusion from the choroid to the inner retina thereby relieving inner retinal hypoxia. This improved oxygenation triggers a two-pronged cascade of events where: (1) constriction of the retinal arteries results in decreased hydrostatic pressure in capillaries and the constriction of capillaries and venules; and (2) the cellular production of VEGF is inhibited. Together, these effects are believed to ultimately result in the inhibition of neovascularization and a decrease in oedema. Cell proliferation and regulation of cellular proteins are induced by the laser photocoagulation, and their therapeutic effect might be an essential part of the physiological response.

However, a complication of laser treatment (either photodynamic laser therapy or laser photocoagulation) is inflammation, leading to further oedema. This may also occur after laser therapy to remove or treat ocular neoplasm. In addition, laser treatment is not always a permanent cure as the blood vessels may begin to grow again, and microaneurysms may reform. Furthermore, laser treatment of abnormal blood vessels cannot be performed on vessels located in certain regions of the retina, such as the central region of the macula.

Therefore, in an embodiment of the description, where laser treatment of the retina is indicted, administration of an anti-oedematous steroid such as a mineralocorticoid may be carried out by injection before or after the laser treatment. Administration of the anti-oedematous steroid may reduce, eliminate or prevent oedema before or after laser therapy and may therefore reduce or eliminate one of the side effects of laser therapy.

In another embodiment the description resides in a use for reducing ocular irritation comprising the step of: administering to a patient a formulation as described above to a patient following corneal surgery (e.g., LASIK® surgery, photorefractive keratectomy (PRK), or other corneal procedures). Preferably the formulation administered to the patient is a compound capable of modulating the activity of mineralocorticoid receptors within cells or tissue located within the eye. Preferably, such mineralocorticoid receptor activity modulating compounds are anti-oedematous steroids or more preferably mineralocorticoids. Such mineralocorticoids reduce or inhibit the exudation of fluids in the eye which may cloud the cornea or the vitreous.

In addition to the other compounds previously described, formulations of the description may be injected with anti-angiogenic agents designed to block the actions of VEGF on endothelial cells in combined therapies. Examples of agents that can be employed in the method of the description are: (a) Lucentis^{®} developed by Genentech; and (b) Macugen^{®} developed by Eyetech Pharmaceuticals. Lucentis^{®} and Macugen^{®} are compounds that are injected into the vitreous and are potent anti-angiogenic compounds. In a highly preferred form, the method of the description is performed by administering a pharmaceutical formulation of a compound capable of modulating the mineralocorticoid receptors within the cells or tissue of the eye as described above and an anti-angiogenic agent such as Lucentis^{®} or Macugen^{®}. Preferably the compound capable of modulating the activity of mineralocorticoid receptors is an anti-oedematous steroid and more preferably a mineralocorticoid as herein described.

Lucentis^{®} (ranibizumab), formerly known as rhuFab V2 or AMD-Fab is a humanized, therapeutic anti-VEGF (vascular endothelial growth factor) antibody fragment developed by Genentech to bind and inhibit VEGF, a protein that plays a critical role in angiogenesis (the formation of new blood vessels) by binding to VEGF and preventing it from interacting with the VEGF receptor on the surface of endothelial cells. Lucentis is designed to block new blood vessel growth and reduce leakage, which are thought to lead to wet AMD disease progression. Lucentis^{®} is injected into the vitreous, and then passes beneath the retina into the area where the abnormal blood vessels are growing. When administered in conjunction with pharmaceutical formulations prepared according to the present description Lucentis should be administered in either about 300 µg or about 500 µg doses for four doses.

Macugen^{®} (pegaptanib sodium, anti-VEGF aptamer or EYE001) developed by Eyetech Pharmaceuticals, consists of a synthetic fragment of genetic material that specifically binds to the VEGF molecule and blocks it from stimulating the receptor on the surface of endothelial cells. When administered in conjunction with pharmaceutical formulations prepared according to the present description Macugen^{®} should be administered in a dose ranging from either about 0.3 mg to about 3.0 mg every four or six weeks.

In another aspect of the description, the use of the description may be performed by administering a pharmaceutical formulation comprising a compound capable of modulating the mineralocorticoid receptors within the cells or tissue of the eye (preferably an anti-oedematous steroid and more preferably a mineralocorticoid as herein described) in combination with a glucocorticoid (e.g. prednisolone, prednisone), an oestrogen (e.g. oestrodiol), an androgen (e.g. testosterone) retinoic acid derivatives (e.g. 9-cis-retinoic acid, 13-trans-retinoic acid, all-trans retinoic acid), a vitamin D derivative (e.g. calcipotriol, calcipotriene), a non-steroidal anti-inflammatory agent, a vitamin D derivative, an anti-infective agent, a protein kinase C inhibitor, a MAP kinase inhibitor, an anti-apoptotic agent, a growth factor, a nutrient vitamin, an unsaturated fatty acid, and/or ocular anti-infective agents, for the treatment of the ophthalmic conditions set forth herein. In still other embodiments of the description, a mixture of these agents may be used.

Ocular anti-infective agents that may be used include, but are not limited to, penicillins (ampicillin, aziocillin, carbenicillin, dicloxacillin, methicillin, nafcillin, oxacillin, penicillin G, piperacillin, and ticarcillin), cephalosporins (cefamandole, cefazolin, cefotaxime, cefsulodin, ceftazidime, ceftriaxone, cephalothin, and moxalactam), aminoglycosides (amikacin, gentamicin, netilmicin, tobramycin, and neomycin), miscellaneous agents such as aztreonam, bacitracin, ciprofloxacin, clindamycin, chloramphenicol, cotrimoxazole, fusidic acid, imipenem, metronidazole, teicoplanin, and vancomycin), antifungals (amphotericin B, clotrimazole, econazole, fluconazole, flucytosine, itraconazole, ketoconazole, miconazole, natamycin, oxiconazole, and terconazole), antivirals (acyclovir, ethyldeoxyuridine, foscarnet, ganciclovir, idoxuridine, trifluridine, vidarabine, and (S)-1-(3-dydroxy-2-phospho-nyluethoxypropyl) cytosine (HPMPC)), antineoplastic agents (cell cycle (phase) nonspecific agents such as alkylating agents (chlorambucil, cyclophosphamide, mechlorethamine, melphalan, and busulfan), anthracycline antibiotics (doxorubicin, daunomycin, and dactinomycin), cisplatin, and nitrosoureas), antimetabolites such as antipyrimidines (cytarabine, fluorouracil and azacytidine), antifolates (methotrexate), antipurines (mercaptopurine and thioguanine), bleomycin, vinca alkaloids (vincrisine and vinblastine), podophylotoxins (etoposide (VP-16)), and nitrosoureas (carmustine, (BCNU)), immunosuppressant agents such as cyclosporin A and SK506, and anti-inflammatory or suppressive agents (inhibitors), and inhibitors of proteolytic enzymes such as plasminogen activator inhibitors. Doses for topical and subconjunctival administration of the above agents, as well as intravitreal dose and vitreous half-life may be found in Intravitreal Surgery Principles and Practice, Peyman G A and Shulman, J Eds., 2nd edition, 1994, Appleton-Longe, the relevant sections of which are expressly incorporated by reference herein.

The present description provides for the use of a compound capable of modulating the activity of mineralocorticoid receptors within cells or tissue located in an eye or adjacent to an eye to treat or prevent an ophthalmic condition in the manufacture of a medicament for the treatment of an ophthalmic condition. The description also provides for the use of an anti-oedematous steroid such as a mineralocorticoid or pharmaceutically acceptable salt thereof as herein described in the manufacture of a medicament for the treatment of an ophthalmic condition.

The use of (a) a compound capable of modulating the activity of mineralocorticoid receptors within cells or tissue located in an eye or adjacent to an eye to treat or prevent an ophthalmic condition; and (b) a second compound chosen from the list comprising: a steroid, heparin, a tetracycline derivative, an anti-prostaglandin, a macrocyclic antibiotic, a metalloproteinase inhibitor or an anti-angiogenic agent designed to block the actions of VEGF on endothelial cells; for the manufacture of a medicament for the treatment of an ophthalmic condition is also provided.

According to a further form, the description provides for the use of compounds capable of modulating the activity of mineralocorticoid receptors in the manufacture of a medicament for the treatment of an exudative retinopathy in an individual.

Further, it provides for use of an effective amount of anti-oedematous steroid or a pharmaceutically acceptable salt thereof in the manufacture of a medicament for the treatment of an exudative retinopathy in an individual. The description also provides for the use of a mineralocorticoid as herein described in the preparation of a medicament for the treatment of exudative retinopathies.

### Examples

Further features of the present invention are more fully described in the following non-limiting Examples. It is to be understood, however, that this detailed description is included solely for the purposes of exemplifying the present invention.

### Example 1

To show the capacity of a mineralocorticoid, specifically fludrocortisone, to down-regulate receptors involved in biochemical pathways associated with oedema and exudative retinopathies, an assay was conducted looking at the activity of VEGF receptors Flk-1 and Flt-1 in choroidal endothelial cells. Flk-1 is known to be involved in cell proliferation whilst Flt-1 is involved in cell permeability.

The expression of Flk-1 and Flt-1 was measured, using fluorescence activated cell sorting (FACS) analysis, in untreated cells, cells treated with phorbolmyristate acetate (PMA), cells treated with PMA and then with the glucocorticoid triamcinolone acetonide (TA) and cells treated with PMA and then with the mineralocorticoid fludrocortisone. PMA is a model for pro-inflammatory cytokines and TA is known to block the glucocorticoid receptor

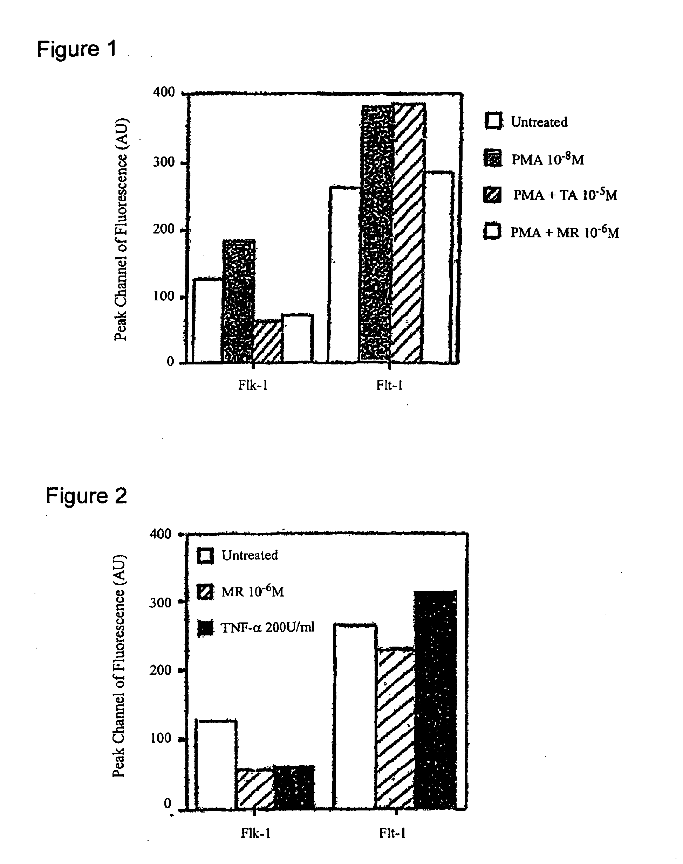

From Figure 1 it can be seen that expression of Flk-1 is increased compared to control cells by administration of PMA. However, this increase is reversed by treatment with fludrocortisone, leading to reduced expression of Flk-1 in fludrocortisone treated cells compared to control (untreated) cells. The TA/Flk-1 pathway is via a combination of direct action on receptor expression and anti-inflammatory effects via the glucocorticoid receptor; noting that VEGF and its receptors mediate pro-inflammatory effects.

Expression of Flt-1 is also increased by administration of PMA, and reduced by treatment with fludrocortisone, with cells treated with fludrocortisone expressing similar levels of Flt-1 as untreated cells.

### Example 2

The expression of Flk-1 and Flt-1 was also investigated in untreated choroidal endothelial cells, choroidal endothelial cells treated with Tumour Necrosis Factor-α (TNF-α) and choroidal endothelial cells treated with the mineralocorticoid fludrocortisone. TNF-α is a pro-inflammatory cytokine.

Expression of FLK-1 is down-regulated from the levels in untreated cells by administration of fludrocortisone. Cells treated with TNF-α express higher levels of Flt-1 than untreated control cells. However, administration of fludrocortisone slightly reduced the expression of Flt-1 compared to control cells

### Example 3

Transendothelial electrical resistance (TER) is a method of measuring cell permeability and thus exudative capacity. High electrical resistance reflects tight cell barriers and low cell permeability. In contrast, no resistance or negative resistance indicates the presence of looser junctions in the cell barrier.

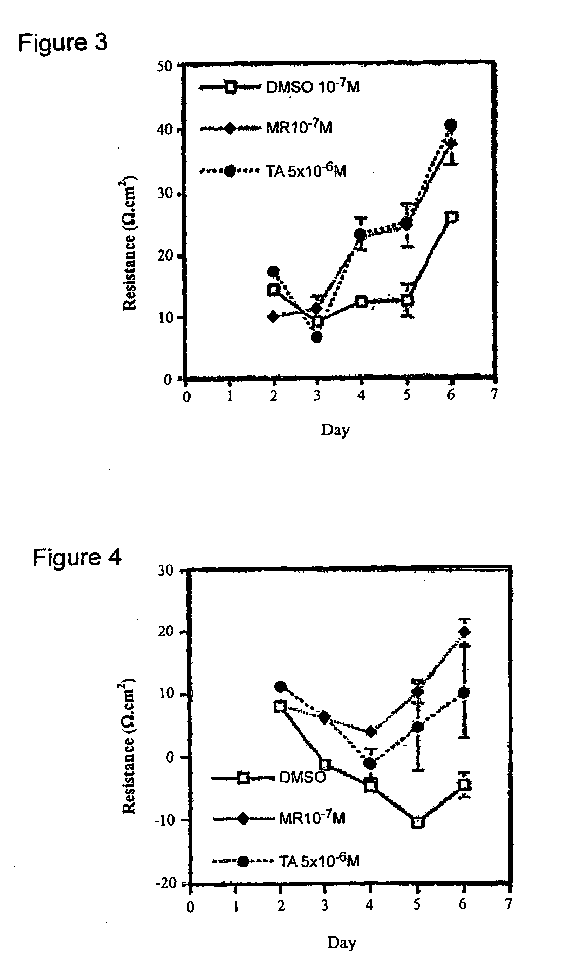

Choroidal endothelial cells treated with dimethyl sulfoxide (DMSO), a surfactant which disrupts the cell membrane, have low electrical resistance, as indicated in Figure 3, compared to cells treated with fludrocortisone or TA. The increased resistance in the presence of fludrocortisone or TA indicates that the steroids aid in the maintenance or repair of the cell membrane barrier, resulting in a reduction in exudation and oedema.

### Example 4

Retinal endothelial cells treated with DMSO exhibit low electrical resistance in contrast to cells treated with fiudrocortisone. Fludrocortisone treated cells exhibit higher resistance than those treated with TA. This indicates that in retinal endothelial cells, fludrocortisone is aiding in the maintenance or repair of the cell membrane barrier and thus reducing exudation and oedema.

### Example 5

Thirty New Zealand albino rabbits weighing between 2.5 and 3.0 kg were used for this study. The rabbits were treated in accordance with the guidelines of the Association for Research in Vision and Ophthalmology (ARVO). One eye of each animal was used for experimental purposes, the other serving as a control eye. Slit lamp and indirect funduscopic examination was performed on all eyes before and after euthanasia.

The rabbits were divided into six groups. Group 1 (n=5) received topical 4 mg/ml deoxycortisone acetate (Spectrum Chemical Corp). Group 2 (n=5) received topical 4 mg/ml 11-deoxycorticosterone (Spectrum Chemical Corp). Group 3 (n=5) received topical 4 mg/ml 11-desoxycortisone (Spectrum Chemical Corp). Group 4 (n=5) received topical 4 mg/ml aldosterone (Farmabios, Italy). Group 5 (n=5) received topical 4 mg/ml deoxycorticosterone (Spectrum Chemical Corp). Group 6 (n=5) received topical fludrocortisone acetate (Farmabios, Italy).

The rabbits were anaesthetised with 1 ml of a mixture of ketamine hydrochloride (35 mg/kg, intramuscular) and xylazine hydrochloride (5 mg/kg, intramuscular). In all groups, drugs were applied topically to the right eye and saline was applied to the left eye four times (at time 0, 1, 2 and 3 hours). Drugs and saline were administered to the eye as a single 50 µL drop.

20 mg/ml dextran-isothiocyanate-fluorescein (FITC-dextran, Sigma) was prepared and a 20 mg/kg dose was injected into the marginal ear vein at time 1.5 hours.

Endotoxin (2.5 µg/kg) isolated from *Salmonella typhimurium* (Sigma) was injected into the same vein at the same time with the third drop occurring at time 2 hours.

The FITC-dextran concentration in the anterior chamber was determined using a prototype fluorophotometer (OcuMetrics, Mountain View, CA) 90 minutes after an endotoxin administration. This instrument has excitation wavelengths of approximately 410 to 490 nm, with emission wavelengths of approximately 520 to 630 nm. The fluorescence emitted by the FITC-dextran was determined in both left and right eyes.

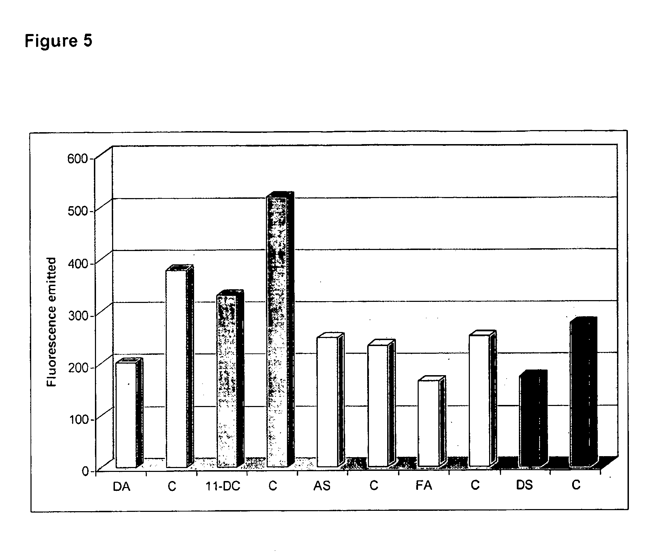

Statistical analysis was performed, comparing the anterior chamber fluorescence measurements of the test and control eye of each animal. A paired Wilcoxon Signed Rank Test (StatXact) was used, with a one sided p-value. The results of this experiment are presented in Figure 5 and Table 1 below.

**Table 1: Anterior Chamber Fluorophotometer Results**

| **Rabbit** | **DA** | **C** | **11-DC** | **C** | **AS** | **C** | **FA** | **C** | **DS** | **C** |
|---|---|---|---|---|---|---|---|---|---|---|
| 1 | 220 | 370 | 696 | 791 | 243 | 281 | 27 | 111 | 340 | 533 |
| 2 | 116 | 312 | 578 | 696 | 75 | 42 | 86 | 162 | 245 | 279 |
| 3 | 455 | 580 | 141 | 457 | 125 | 224 | 261 | 316 | 59 | 283 |
| 4 | 100 | 380 | 147 | 457 | 540 | 324 | 445 | 639 | 86 | 112 |
| 5 | 113 | 253 | 92 | 194 | 256 | 300 | 10 | 30 | 140 | 176 |
| Mean | 201 | 379 | 331 | 519 | 248 | 234 | 166 | 252 | 174 | 277 |

| | | | | | | | | | | |
|---|---|---|---|---|---|---|---|---|---|---|
| C = control; DA = deoxycorticosterone acetate; 11-DC = 11-desoxycortisone; AS = aldosterone; FA = fludrocortisone acetate; DS= deoxycorticosterone | | | | | | | | | | |

Administration of 4 mg/ml 11-desoxycortisone gave a 37% reduction in the exudative response (p<0.03), 4 mg/ml deoxycorticosterone acetate gave a 47% reduction in the exudative response (p<0.03), 4 mg/ml fludrocortisone acetate gave a 34% reduction in the exudative response (p<0.03), 4 mg/ml deoxycorticosterone gave a 37% reduction in the exudative response (p<0.03). The results for aldosterone were very variable and those for 11-deoxycorticosterone did not show a reduction in the exudative response.

### Example 6

To determine if mineralocorticoids are active in the regulation of genes controlling exudation in retinal cells, specifically retinal pigment epithelial (RPE) cells and choroidal endothelial (CE) cells, real time competitive PCR was performed on both mouse and non-human primate cells at early passage.

Real-Time comparative PCR was performed using a 96-well microtiter plate format on an ABI PRISM 7700 Sequence Detection System (PE Applied Biosystems) equipped with a Sequence Detection System (SDS) software version 1.6.3.

PCR was performed using 250 ng of cDNA of each sample, specific for the selected gene probes and Taqman PCR master mix and the method follows the manufacturer's protocol with slight modifications. The primers and probes for both mouse and human genes associated with angiogenesis and oedema were obtained from Taqman, Assays On Demand, PE Applied Biosystems, USA. Quantum RNA classic II 18S Internal Standard (Ambion) were used as an endogenous control. An 18S primer-competimer ratio of 1:4 was used in all experiments.

To standardise and evaluate RPE and CE gene expression, aliquots of cDNA (250 ng) prepared from the retinal pigment epithelial cells and choroidal endothelial cells were used as templates in all PCR reactions. All experiments were carried out in triplicate. The RT-PCR cycles were 55°C for 2 minutes, 95°C for 10 minutes and 40 cycles of 95°C for 30 sec, 60°C for 30 sec and 72°C for 2 minutes.

The comparative quantification values were obtained from the threshold cycle (C_{T}) number at which an increase in signal (associated with an exponential growth of PCR products) is detected using SDS software. Three PCRs were carried out (n = 3) for each sample/primer set, and the mean experimental value was used as the relative quantification value. For all probes, amplification specificity was confirmed by the manufacturer and the reaction products were separated on 3% agarose gel and stained with ethidium bromide for visual confirmation of PCR products.

### • Data Analysis by Comparative C_{T} (ΔΔC_{T}) Method

The C_{T} value represents the PCR cycle at which an increase in reporter fluorescence (ΔRn) above the line of the optimal value (optimal ΔRn) is first detected. The calculation for comparative C_{T} (ΔΔC_{T}) method (Brink N et al. Comparative quantification of IL-1β, IL-10, II-10r, TNFα and IL-7 mRNA levels in UV-irradiated human skin in vivo. Inflamm Res. 2000; 49:290-296). Average C_{T} value of all genes and 18S rRNA is calculated from each of the triplicate repeats and this is applied for all different experiments. Finally the mean C_{T} value is calculated from three average C_{T} values for each cell sample experiment. The ΔC_{T} value is determined by subtracting the corresponding mean 18S Ct value from the mean MR1 Ct value. The standard deviation of the difference is calculated from the standard deviations of the gene of interest and the corresponding 18S values. The ΔΔC_{T} value is obtained by subtracting the ΔC_{T} reference value from the ΔC_{T} sample value, this is known as the 2^{-ΔΔC}_{T} method. This is the subtraction of an arbitrary constant, so the standard deviation of the ΔΔC_{T} is similar to the standard deviation of the ΔC_{T} value. The value given for the gene of interest relative to their corresponding normal, untreated, value will be determined by evaluating the expression by 2^{-ΔΔC}_{T}. as described above.

### Example 7

PDT-induced oedema is induced in Dutch belted rabbits (n=6) using verteporfin as stated in Burke et al, (2004) ARVO abstract.

The PDT procedure consists of an intravenous infusion of Visudyne (10mls or 0.2mg/kg) at a rate of 1ml/min for 10 min. Then, 20 -25 minutes post-verteporfin injection, two ~1.5mm spots are induced at the posterior pole using PDT laser treatment, 689 nm for 83 seconds at 600mW/cm² 50J/cm² (Burke et al., 2004).

One to two hours after PDT treatment, intravitreal injection of the mineralocorticoid (100-200µg in 0.1 ml) is carried out. One eye of each animal serves as the "test" eye and the other eye serves as the "control" eye. The test eye receives 0.1 ml of the mineralocorticoid intravitreally, while the control eye receives a 0.1ml intravitreal injection of BSS. Oedema is assessed at 12 and 24 hours and days 3 and 6-9 post-treatment.

Assessment is performed using fluorescein angiography, fundus examination and OCT measurement of retinal depth.

## Claims

1. Use of a therapeutically effective amount of a compound capable of modulating the activity of mineralocorticoid receptors within cells or tissue located in an eye or adjacent to an eye in the individual to be treated, wherein said compound is selected from the group consisting of the mineralocorticoids: aldosterone, fludrocortisone, fludrocortisone acetate; deoxycorticosterone; 11-desoxycortisone; or deoxycorticosterone acetate or a pharmaceutically acceptable salt thereof in the manufacture of a composition for the prophylactic or therapeutic treatment of an individual with an exudative retinopathy.

2. A compound selected from the group consisting of the mineralocorticoids: aldosterone, fludrocortisone, fludrocortisone acetate; deoxycorticosterone; 11-desoxycortisone;. or deoxycorticosterone acetate or a pharmaceutically acceptable salt thereof for use in the prophylactic or therapeutic treatment of an individual with an exudative retinopathy.

3. The use of claim 1 or the compound for use according to claim 2, wherein the compound is sparingly soluble in the vitreous.

4. The use of claims 1 or 3 or the compound for use according to claims 2 or 3, wherein said cells or tissue are selected from the group consisting of: retinal cells, retinal pigment epithelial cells, the epithelial cells of the BRB, or choroidal endothelial cells.

5. The use of any of claims 1 or 3 to 4 or the compound for use according to any of claims 2 to 4 for the prophylactic treatment of an individual with an exudative retinopathy in a first eye but not a second eye, wherein the compound is to be administered to the second eye.

6. The use of any of claims 1 or 3 to 5 or the compound for use according to any of claims 2 to 5, wherein the compound is to be administered by intravitreal injection to said individual.

7. The use of any of claims 1 or 3 to 6 or the compound for use according to any of claims 2 to 6, wherein more than one mineralocorticoid is to be administered to said individual.

8. The use of any of claims 1 or 3 to 7 or the compound for use according to any of claims 2 to 7, wherein the mineralocorticoid is to be deposited intravitreally and the dosage is between about 1 mg and about 8 mg.

9. The use of any of claims 1 or 3 to 8 or the compound for use according to any of claims 2 to 8, wherein the dosage is about 4 mg of mineralocorticoid.

10. A pharmaceutical composition for use in the treatment or prophylaxis of an exudative retinopathy comprising a therapeutically effective amount of a compound capable of modulating the activity of mineralocorticoid receptors within cells or tissues located in the eye, wherein said compound is selected from the group consisting of the mineralocorticoids: aldosterone, fludrocortisone, fludrocortisone acetate; deoxycorticosterone; 11 desoxycortisone; or deoxycorticosterone acetate or a pharmaceutically acceptable salt thereof.

11. The composition for use of claim 10 further comprising a pharmaceutically acceptable additive.

12. The pharmaceutical composition for use according to claim 11, wherein the pharmaceutically acceptable additive is selected from the group consisting of a carrier; an adjunct; an excipient; or a non-toxic, non-therapeutic, non-immunogenic stabilizer.

13. The composition for use according to claim 11 or claim 12, wherein the pharmaceutically acceptable additive is compatible with the vitreous and does not leave any vision impairing residue in the eye.

14. The composition for use according to any of claims 11 to 13, wherein the pharmaceutically acceptable additive used in the composition is suited to the delivery of said pharmaceutical composition as an intravitreal depot injection.

15. The composition for use according to any of claims 11 to 13, wherein the pharmaceutically acceptable additive used in the composition is suited to the delivery of said pharmaceutical composition as an ointment, a gel, a mist or eye drops.

16. The pharmaceutical composition for use according to any of claims 10 to 15 for use in the treatment or prophylaxis of an exudative retinopathy.

17. Use of the pharmaceutical composition according to any of claims 10 to 15 in the manufacture of a composition for the prophylactic or therapeutic treatment of an individual with an exudative retinopathy.

18. The pharmaceutical composition according to any of claims 12 to 17 for use in the treatment or prophylaxis of an exudative retinopathy.

19. The use of any of claims 1, 3 to 9 or 17 or the compound for use any of claims 2 to 9, comprising the further step of: administering laser treatment to the retina.

20. The use of any of claims 1, 3 to 9 or 17 or the compound for use any of claims 2 to 9, comprising the further step of: performing one or more other therapies selected from the list comprising: photodynamic therapy, laser surgery (eg LASIK, LASEK, refractive laser
treatment), laser photocoagulation or one or more biological or pharmaceutical treatments.

21. The use or the compound for use according to claim 20, wherein the laser treatment is treatment of the retina and administration of the compound capable of modulating the activity of mineralocorticoid receptors is to be carried out by injection after the laser treatment.

22. The use or the compound for use according to claim 20, wherein the laser treatment is treatment of the retina and administration of the compound capable of modulating the activity of mineralocorticoid receptors is to be carried out by injection before the laser treatment.

## Patentansprüche

1. Verwendung einer therapeutisch wirksamen Menge einer Verbindung, die fähig ist, die Aktivität von Mineralcorticoidrezeptoren in Zellen oder Gewebe, angeordnet in einem Auge oder in der Nähe eines Auges des zu behandelnden Subjekts, zu modulieren, wobei die Verbindung ausgewählt ist aus der Gruppe bestehend aus den Mineralcorticoiden: Aldosteron; Fludrocortison; Fludrocortisonacetat; Desoxycorticosteron; 11-Desoxycortison; und Desoxycorticosteronacetat oder ein pharmazeutisch verträgliches Salz davon, bei der Herstellung einer Zusammensetzung für die prophylaktische oder therapeutische Behandlung eines Individuums mit exudativer Retinopathie.

2. Verbindung, ausgewählt aus der Gruppe bestehend aus den Mineralcorticoiden: Aldosteron; Fludrocortison; Fludrocortisonacetat; Desoxycorticosteron; 11-Desoxycortison; und Desoxycorticosteronacetat oder ein pharmazeutisch verträgliches Salz davon, für die Verwendung bei der prophylaktischen oder therapeutischen Behandlung eines Individuums mit exudativer Retinopathie.

3. Verwendung gemäß Anspruch 1 oder Verbindung für die Verwendung gemäß Anspruch 2, wobei die Verbindung im Glaskörper schwach löslich ist.

4. Verwendung gemäß Anspruch 1 oder 3 oder Verbindung für die Verwendung gemäß Anspruch 2 oder 3, wobei die Zellen oder das Gewebe ausgewählt sind aus der Gruppe bestehend aus: Retinalzellen, Retinalpigmentepithelzellen, den Epithelzellen des BRB und Choroidalepithelzellen.

5. Verwendung gemäß einem der Ansprüche 1 oder 3 bis 4 oder Verbindung für die Verwendung gemäß einem der Ansprüche 2 bis 4 für die prophylaktische Behandlung eines Individuums mit einer exudativen Retinopathie in einem ersten Auge, aber nicht in einem zweiten Auge, wobei die Verbindung an das zweite Auge zu verabreichen ist.

6. Verwendung gemäß einem der Ansprüche 1 oder 3 bis 5 oder Verbindung für die Verwendung gemäß einem der Ansprüche 2 bis 5, wobei die Verbindung durch intravitreale Injektion an das Individuum zu verabreichen ist.

7. Verwendung gemäß einem der Ansprüche 1 oder 3 bis 6 oder Verbindung für die Verwendung gemäß einem der Ansprüche 2 bis 6, wobei mehr als ein Mineralcorticoid an das Individuum zu verabreichen ist.

8. Verwendung gemäß einem der Ansprüche 1 oder 3 bis 7 oder Verbindung für die Verwendung gemäß einem der Ansprüche 2 bis 7, wobei das Mineralcorticoid intravitreal zu deponieren ist und die Dosierung zwischen etwa 1 mg und etwa 8 mg beträgt.

9. Verwendung gemäß einem der Ansprüche 1 oder 3 bis 8 oder Verbindung für die Verwendung gemäß einem der Ansprüche 2 bis 8, wobei die Dosierung etwa 4 mg an Mineralcorticoid beträgt.

10. Pharmazeutische Zusammensetzung für die Verwendung bei der Behandlung oder Prophylaxe einer exudativen Retinopathie, umfassend eine therapeutisch wirksame Menge einer Verbindung, die fähig ist, die Aktivität von Mineralcorticoidrezeptoren in Zellen oder Gewebe, angeordnet in dem Auge, zu modulieren, wobei die Verbindung ausgewählt ist aus der Gruppe bestehend aus den Mineralcorticoiden: Aldosteron, Fludrocortison, Fludrocortisonacetat; Desoxycorticosteron; 11-Desoxycortison; und Desoxycorticosteronacetat oder ein pharmazeutisch verträgliches Salz davon.

11. Zusammensetzung für die Verwendung gemäß Anspruch 10, ferner umfassend einen pharmazeutisch verträglichen Zusatzstoff.

12. Pharmazeutische Zusammensetzung für die Verwendung gemäß Anspruch 11, wobei der pharmazeutisch verträgliche Zusatzstoff ausgewählt ist aus der Gruppe bestehend aus: einem Träger; einem Hilfsstoff; einem Exzipienten; einem nichttoxischen nichttherapeutischen nichtimmunogenen Stabilisator.

13. Zusammensetzung für die Verwendung gemäß Anspruch 11 oder Anspruch 12, wobei der pharmazeutisch verträgliche Zusatzstoff mit dem Glaskörper verträglich ist und keinen sehbeeinträchtigenden Rückstand im Auge zurücklässt.

14. Zusammensetzung für die Verwendung gemäß einem der Ansprüche 11 bis 13, wobei der in der Zusammensetzung verwendete pharmazeutisch verträgliche Zusatzstoff für die Abgabe der pharmazeutischen Zusammensetzung als intravitreale Depotinjektion geeignet ist.

15. Zusammensetzung für die Verwendung gemäß einem der Ansprüche 11 bis 13, wobei der in der Zusammensetzung verwendete pharmazeutisch verträgliche Zusatzstoff für die Abgabe der pharmazeutischen Zusammensetzung als Salbe, als Gel, als Nebel oder Augentropfen geeignet ist.

16. Pharmazeutische Zusammensetzung für die Verwendung gemäß einem der Ansprüche 10 bis 15 für die Verwendung bei der Behandlung oder Prophylaxe einer exudativen Retinopathie.

17. Verwendung der pharmazeutischen Zusammensetzung gemäß einem der Ansprüche 10 bis 15 bei der Herstellung einer Zusammensetzung für die prophylaktische oder therapeutische Behandlung eines Individuums mit einer exudativen Retinopathie.

18. Pharmazeutische Zusammensetzung gemäß einem der Ansprüche 12 bis 17 für die Verwendung bei der Behandlung oder Prophylaxe einer exudativen Retinopathie.

19. Verwendung gemäß einem der Ansprüche 1, 3 bis 9 oder 17 oder Verbindung für die Verwendung gemäß einem der Ansprüche 2 bis 9, umfassend den weiteren Schritt der: Verabreichung einer Laserbehandlung an die Retina.

20. Verwendung gemäß einem der Ansprüche 1, 3 bis 9 oder 17 oder Verbindung für die Verwendung gemäß einem der Ansprüche 2 bis 9, umfassend den weiteren Schritt der: Durchführung einer oder mehrerer anderer Therapien, ausgewählt aus der Liste, umfassend: photodynamische Therapie, Laserchirurgie (beispielsweise LASIK, LASEK, refraktive Laserbehandlung), Laser-Photokoagulation oder eine oder mehrere biologische oder pharmazeutische Behandlungen.

21. Verwendung der Verbindung für die Verwendung gemäß Anspruch 20, wobei die Laserbehandlung eine Behandlung der Retina ist und die Verabreichung der Verbindung, die fähig ist, die Aktivität von Mineralcorticoidrezeptoren zu modulieren, durch Injektion nach der Laserbehandlung durchzuführen ist.

22. Verwendung der Verbindung für die Verwendung gemäß Anspruch 20, wobei die Laserbehandlung eine Behandlung der Retina ist und die Verabreichung der Verbindung, die fähig ist, die Aktivität von Mineralcorticoidrezeptoren zu modulieren, durch Injektion vor der Laserbehandlung durchzuführen ist.

## Revendications

1. Utilisation d'une quantité thérapeutiquement efficace d'un composé capable de moduler l'activité de récepteurs de minéralocorticoïde dans des cellules ou un tissu situés dans un oeil ou adjacents à un oeil chez l'individu à traiter, ledit composé étant choisi dans le groupe constitué des minéralocorticoïdes :
aldostérone, fludrocortisone, acétate de fludrocortisone ; désoxycorticostérone ; 11-désoxycortisone ; ou acétate de désoxycorticostérone ou
un sel pharmaceutiquement acceptable de ceux-ci dans la fabrication d'une composition pour le traitement prophylactique ou thérapeutique d'un individu ayant une rétinopathie exsudative.

2. Composé choisi dans le groupe constitué des minéralocorticoïdes : aldostérone, fludrocortisone, acétate de fludrocortisone ; désoxycorticostérone ; 11-désoxycortisone ; ou acétate de désoxycorticostérone ou un sel pharmaceutiquement acceptable de ceux-ci pour utilisation dans le traitement prophylactique ou thérapeutique d'un individu ayant une rétinopathie exsudative.

3. Utilisation de la revendication 1 ou composé pour utilisation selon la revendication 2, le composé étant faiblement soluble dans le corps vitré.

4. Utilisation des revendications 1 ou 3 ou composé pour utilisation selon les revendications 2 ou 3, lesdits cellules ou tissu étant choisis dans le groupe constitué de : cellules rétiniennes, cellules épithéliales du pigment rétinien, cellules épithéliales du BRB, ou cellules endothéliales choroïdiennes.

5. Utilisation de l'une quelconque des revendications 1 ou 3 à 4 ou composé pour utilisation selon l'une quelconque des revendications 2 à 4 pour le traitement prophylactique d'un individu ayant une rétinopathie exsudative dans un premier oeil mais pas un second oeil, le composé étant destiné à être administré au second oeil.

6. Utilisation de l'une quelconque des revendications 1 ou 3 à 5 ou composé pour utilisation selon l'une quelconque des revendications 2 à 5, le composé étant destiné à être administré par injection intravitréenne audit individu.

7. Utilisation de l'une quelconque des revendications 1 ou 3 à 6 ou composé pour utilisation selon l'une quelconque des revendications 2 à 6, plus d'un minéralocorticoïde étant destiné à être administré audit individu.

8. Utilisation de l'une quelconque des revendications 1 ou 3 à 7 ou composé pour utilisation selon l'une quelconque des revendications 2 à 7, le minéralocorticoïde étant destiné à être déposé de façon intravitréenne et la dose étant comprise entre environ 1 mg et environ 8 mg.

9. Utilisation de l'une quelconque des revendications 1 ou 3 à 8 ou composé pour utilisation selon l'une quelconque des revendications 2 à 8, la dose étant d'environ 4 mg de minéralocorticoïde.

10. Composition pharmaceutique pour utilisation dans le traitement ou la prophylaxie d'une rétinopathie exsudative comprenant une quantité thérapeutiquement efficace d'un composé capable de moduler l'activité de récepteurs de minéralocorticoïde dans des cellules ou des tissus situés dans l'oeil, ledit composé étant choisi dans le groupe constitué des minéralocorticoïdes : aldostérone, fludrocortisone, acétate de fludrocortisone ; désoxycorticostérone ; 11-désoxycortisone ; ou acétate de désoxycorticostérone ou un sel pharmaceutiquement acceptable de ceux-ci.

11. Composition pour utilisation selon la revendication 10 comprenant en outre un additif pharmaceutiquement acceptable.

12. Composition pharmaceutique pour utilisation selon la revendication 11, l'additif pharmaceutiquement acceptable étant choisi dans le groupe constitué d'un véhicule ; un adjuvant ; un excipient ; ou un stabilisant non toxique, non thérapeutique, non immunogène.

13. Composition pour utilisation selon la revendication 11 ou la revendication 12, l'additif pharmaceutiquement acceptable étant compatible avec le corps vitré et ne laissant aucun résidu altérant la vision dans l'oeil.

14. Composition pour utilisation selon l'une quelconque des revendications 11 à 13, l'additif pharmaceutiquement acceptable utilisé dans la composition étant adapté pour l'administration de ladite composition pharmaceutique sous la forme d'une injection de dépôt intravitréenne.

15. Composition pour utilisation selon l'une quelconque des revendications 11 à 13, l'additif pharmaceutiquement acceptable utilisé dans la composition étant adapté pour l'administration de ladite composition pharmaceutique sous la forme d'une pommade, un gel, un brouillard ou des gouttes ophtalmiques.

16. Composition pharmaceutique pour utilisation selon l'une quelconque des revendications 10 à 15 pour utilisation dans le traitement ou la prophylaxie d'une rétinopathie exsudative.

17. Utilisation de la composition pharmaceutique selon l'une quelconque des revendications 10 à 15 dans la fabrication d'une composition pour le traitement prophylactique ou thérapeutique d'un individu ayant une rétinopathie exsudative.

18. Composition pharmaceutique selon l'une quelconque des revendications 12 à 17 pour utilisation dans le traitement ou la prophylaxie d'une rétinopathie exsudative.

19. Utilisation de l'une quelconque des revendications 1, 3 à 9 ou 17 ou composé pour utilisation selon l'une quelconque des revendications 2 à 9, comprenant l'étape supplémentaire de : administration d'un traitement laser à la rétine.

20. Utilisation de l'une quelconque des revendications 1, 3 à 9 ou 17 ou composé pour utilisation selon l'une quelconque des revendications 2 à 9, comprenant l'étape supplémentaire de : conduite d'une ou plusieurs autres thérapies choisies dans la liste comprenant : la thérapie photodynamique, la chirurgie laser (par exemple, LASIK, LASEK, un traitement laser réfractif), la photocoagulation au laser ou un ou plusieurs traitements biologiques ou pharmaceutiques.

21. Utilisation ou composé pour utilisation selon la revendication 20, le traitement laser étant un traitement de la rétine et l'administration du composé capable de moduler l'activité de récepteurs de minéralocorticoïde étant destinée à être effectuée par injection après le traitement laser.

22. Utilisation ou composé pour utilisation selon la revendication 20, le traitement laser étant un traitement de la rétine et l'administration du composé capable de moduler l'activité de récepteurs de minéralocorticoïde étant destinée à être effectuée par injection avant le traitement laser.
